(19) Europäisches Patentamt
European Patent Office
Office européen des brevets

(11) Veröffentlichungsnummer: **0 212 360**
**B1**

(12) # EUROPÄISCHE PATENTSCHRIFT

(45) Veröffentlichungstag der Patentschrift: **08.11.89**

(21) Anmeldenummer: **86110445.3**

(22) Anmeldetag: **29.07.86**

(51) Int. Cl.⁴: **C 07 D 413/12**, C 07 D 417/12, C 07 D 409/12, C 07 D 401/12, C 07 D 405/12, C 07 D 403/12, A 01 N 43/56

(54) **Substituierte Pyrazolin-5-one.**

(30) Priorität: **10.08.85 DE 3528753**

(43) Veröffentlichungstag der Anmeldung: **04.03.87 Patentblatt 87/10**

(45) Bekanntmachung des Hinweises auf die Patenterteilung: **08.11.89 Patentblatt 89/45**

(84) Benannte Vertragsstaaten: **AT BE CH DE FR GB IT LI NL**

(56) Entgegenhaltungen:
**EP-A- 0 048 911**
**EP-A- 0 096 903**
**EP-A- 0 166 171**
**US-A- 2 510 696**

**Arch. Pharm. (1976) 309, 900-908**
**Chem. Abstracts (1909) 3, 536-537**
**Chemical Abstracts, Band 97, Nr. 5, 2. August 1982, Columbus, Ohio, USA UEDA, TAISEJ;"Synthesis of pyrazolone derivatives. XXXX. Synthesis and analgesic activity of 4-alkoxyimino(or alkylaminomethylene)-3-methylcarbamoyl-1-methyl(or phenyl)-2-pyrazolin-5-ones." Seite 569, Spalte 2, Zusammenfassung-Nr. 38 878h**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Jelich, Klaus, Dr., Pahlkestrasse 5, D-5600 Wuppertal 1 (DE)**
Erfinder: **Brandes, Wilhelm, Dr., Eichendorffstrasse 3, D-5653 Leichlingen 1 (DE)**
Erfinder: **Hänssler, Gerd, Dr., Am Arenzberg 58a, D-5090 Leverkusen 3 (DE)**
Erfinder: **Reinecke, Paul, Dr., Steinstrasse 8, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 212 360 B1

**Beschreibung**

Die Erfindung betrifft neue substituierte Pyrazolin-5-one, mehrere Verfahren zu ihrer Herstellung und ihre Verwendung als Schädlingsbekämfungsmittel.

Es ist bereits bekannt, daß organische Stickstoff-Verbindungen wie beispielsweise das Zink-ethylen-1,2-bis-(dithiocarbamat) fungizide Eigenschaften besitzen (vgl. z.B. R. Wegler «Chemie der Pflanzenschutz- und Schädlingsbekämpfungsmittel», Springer Verlag Berlin, Heidelberg, New York 1970, Band 2, S 65 f).

Ferner sind 4-Hydroximino-pyrazolin-5-one als Fungizide bekannt (vgl. US-A 2 510 696).

Weitere 4-Oximino-pyrazolin-5-one sind zur Bekämpfung von Schädlingen, vor allem Pilzen beschrieben (vgl. EP-A-166 171).

Die Wirkung dieser Verbindungen ist jedoch insbesondere bei niedrigen Aufwandmengen und -konzentrationen nicht immer in allen Anwendungsbereichen völlig zufriedenstellend.

Es wurden neue substituierte Pyrazolin-5-one der allgemeinen Formel (I)

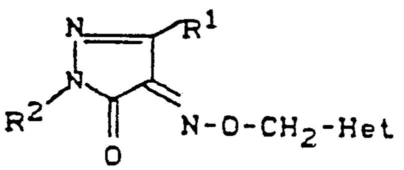

(I)

in welcher

$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Het für einen gegebenenfalls ein- bis dreifach, gleich oder verschieden substituierten und/oder benzannellierten Heterocyclus der Formel

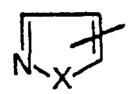 ; 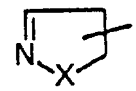 ; 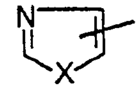 ;

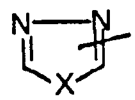 ; 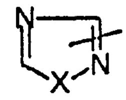 ; 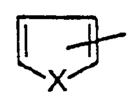 ;

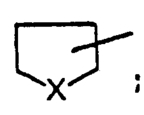 ; 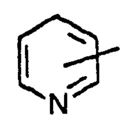 ; 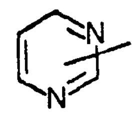 ;

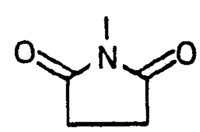 oder 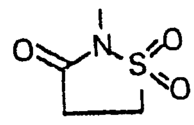

steht, wobei als Substituenten jeweils in Frage kommen: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyrazolin-5-one der allgemeinen Formel (I)

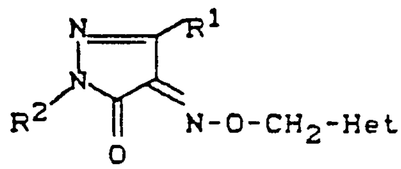

(I)

in welcher

$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy

und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Het für einen gegebenenfalls ein- bis dreifach, gleich oder verschieden substituierten und/oder benzannellierten Heterocyclus der Formel

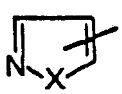 ; 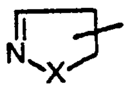 ; 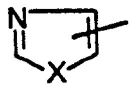 ;

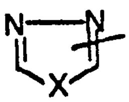 ; 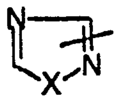 ; 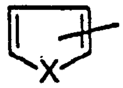 ;

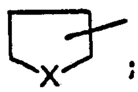 ; 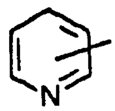 ; 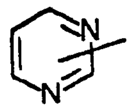 ;

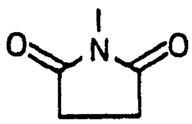 oder 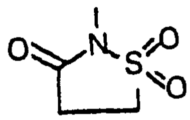

steht, wobei als Substituenten jeweils in Frage kommen: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht,

erhält, wenn man

(a) 4-Oximino-pyrazolin-5-one der Formel (II)

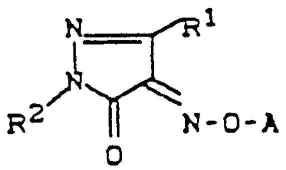 (II)

in welcher

$R^1$ und $R^2$ die oben angegebene Bedeutung haben und
A für Wasserstoff oder ein Alkalimetallkation steht,

mit Alkylierungsmitteln der Formel (III)

$$Het\text{-}CH_2\text{-}X' \qquad (III)$$

in welcher

Het die oben angegebene Bedeutung hat und

X' für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Katalysators umsetzt, oder wenn man

(b) Alkoximinocarbonsäureester der Formel (IV),

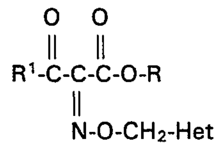 (IV)

in welcher

R für Alkyl steht und

$R^1$ und Het die oben angegebene Bedeutung haben,

mit Hydrazin-Derivaten der Formel (V)

$$R^2\text{-}NH\text{-}NH_2 \qquad (V)$$

in welcher

$R^2$ die oben angegebene Bedeutung hat

gegebenenfalls in Gegenwart eines Verdünnungsmittels umsetzt,
oder wenn man

(c) die nach Verfahren (a) oder nach Verfahren (b) erhältlichen 4-Alkoximino-pyrazolin-5-one der Formel (Ia),

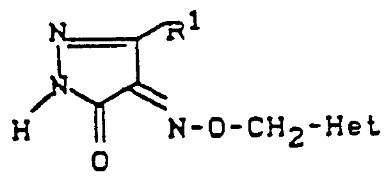 (Ia)

in welcher

$R^1$ und Het die oben angegebene Bedeutung haben, mit Alkylierungsmitteln der Formel (VI)

$$R^{2'}\text{-}Y \qquad (VI)$$

in welcher

$R^{2'}$ für jeweils geradkettiges oder verzweigtes Alkyl, Alkenyl, Alkinyl, Cyanalkyl, Hydroxyalkyl, Alkoxyalkyl, Alkylthioalkyl, Hydroxycarbonylalkyl, Alkoxycarbonylalkyl mit jeweils bis zu 8 Kohlenstoffatomen in den einzelnen Alkyl- bzw. Alkenyl- oder Alkinylteilen, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen in Arylteil, wobei als Substituenten in dem Arylteil jeweils in Frage kommen:
Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Y für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen substituierten Pyrazolin-5-one der Formel (I) fungizide und bakterizide Eigenschaften besitzen.

Überraschenderweise zeigen die neuen substituierten Pyrazolin-5-one der Formel (I) bessere fungizide Eigenschaften als das aus dem Stand der Technik bekannte Zinkethylen-1,2-bis-dithiocarbamat, welches wirkungsmäßig eine naheliegende Verbindung ist.

Die erfindungsgemäßen substituierten Pyrazolin-5-one sind durch die Formel (I) allgemein definiert. Bevorzugt sind Verbindungen der Formel (I), bei welchen

$R^1$ und $R^2$ unabhängig voneinander jeweils für Wasserstoff, Methyl, Ethyl, n- oder i-Propyl, n-, i-, s- oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für gegebenenfalls ein- bis dreifach, gleich oder verschieden substituiertes Phenyl oder Benzyl stehen, wobei als Substituenten in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

Het für einen gegebenenfalls ein- bis dreifach, gleich oder verschieden substituierten und/oder benzannellierten Heterocyclus der Formel

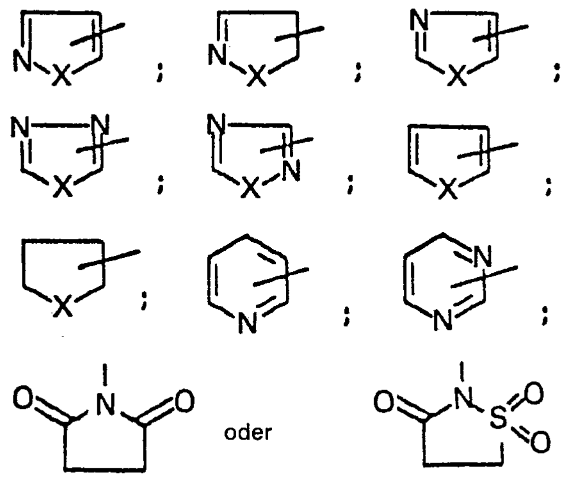
; ; ; ; ; ; ; ; ; oder

steht, wobei als Substituenten jeweils in Frage kommen: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht.

Im einzelnen seien die bei den Herstellungsbeispielen aufgeführten Verbindungen namentlich genannt.

Verwendet man als Ausgangsstoffe beispielsweise 4-Hydroximino-1,3-dimethyl-pyrazolin-5-on und 3-Methyl-5-chlormethyl-1,2-oxazol, so läßt sich der Reaktionsablauf des erfindungsgemäßen Verfahrens (a) durch das folgende Formelschema darstellen:

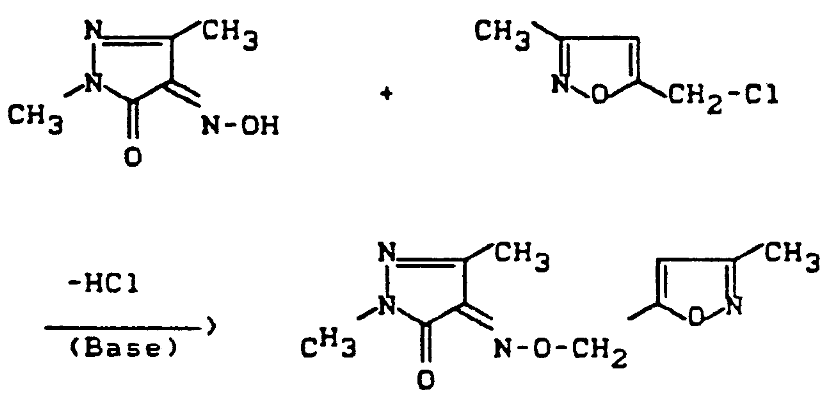


Verwendet man als Ausgangsstoffe beispielsweise β-Keto-α-[(3-ethylisoxazol-5-yl)-methoximino]-buttersäureethylester und Hydrazinhydrat, so läßt sich der Reaktionsablauf des erfindungsgemäßen Verfahrens (b) durch das folgende Formelschema darstellen:

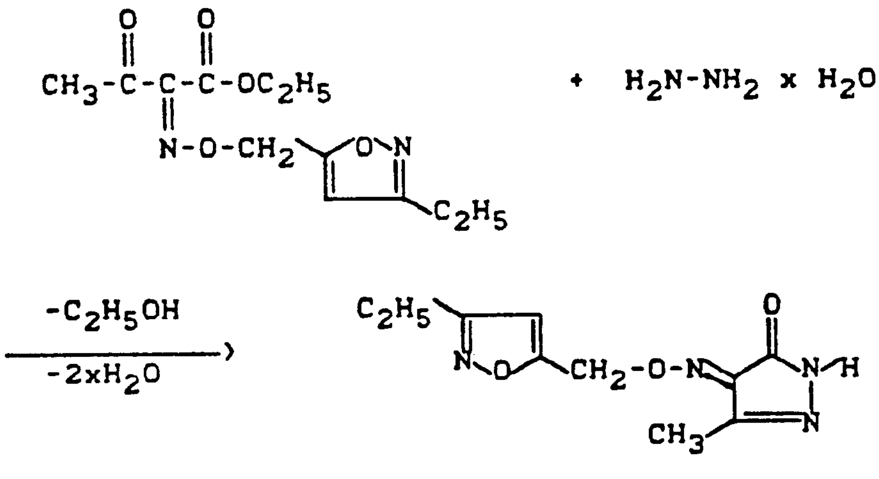


Verwendet man als Ausgangsstoffe beispielsweise 4-(Benzoxazol-2-ylmethyl-oximino)-3-methyl--pyrazolin-5-on und Chloracetonitril, so läßt sich der Reaktionsablauf des erfindungsgemäßen Verfahrens (c) durch das folgende Formelschema darstellen:

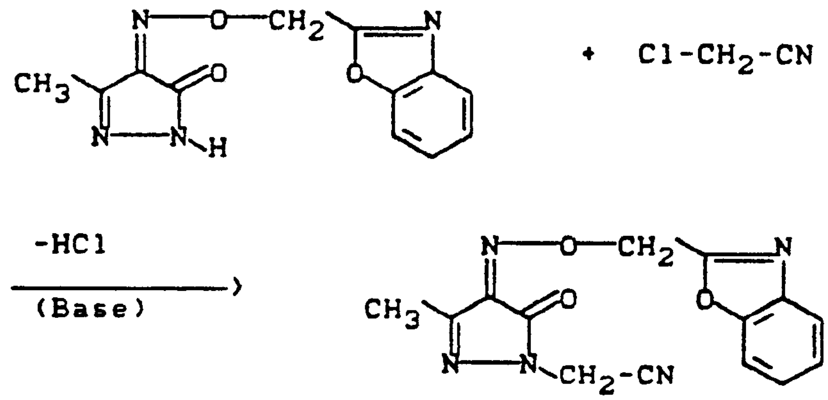


Die zur Durchführung des erfindungsgemäßen Verfahrens (a) als Ausgangsstoffe benötigten 4-Oximino-pyrazolin-5-one sind durch die Formel (II) allgemein definiert. In dieser Formel (II) stehen $R^1$ und $R^2$ vorzugsweise für diejenigen Reste, die schon bei der Beschreibung der erfindungsgemäßen Stoffe der Formel (I) für diese Substituenten genannt wurden. A steht vorzugsweise für Wasserstoff oder für ein Natrium- oder Kaliumkation.

Die 4-Oximino-pyrazolin-5-one der Formel (II) sind teilweise bekannt [vgl. z.B. Ber. dtsch. chem. Ges. 29, 249 (1986); Coll. Czech. Chem. Commun. 25, 55 (1960); Arch. Pharm. 309, 900 (1976); Liebigs Ann. Chem. 1976, 1380].

Man erhält sie beispielsweise, wenn man β-Ketoester der Formel (VII)

$$R^1-\underset{\underset{O}{\|}}{C}-CH_2-COOR^4 \qquad (VII)$$

in welcher

$R^1$ die oben angegebene Bedeutung hat und

$R^4$ für niederes Alkyl, insbesondere für Methyl oder Ethyl steht,

oder wenn man Ethoxymethylenmalonester der Formel (VIII)

$$C_2H_5O-CH=C\begin{matrix}CO_2C_2H_5\\ CO_2C_2H_5\end{matrix} \qquad (VIII)$$

zunächst in einer 1. Stufe mit Hydrazinen der Formel (V)

$$R^2-NH-NH_2 \qquad (V)$$

in welcher

$R^2$ die oben abgegebene Bedeutung hat,

gegebenenfalls in Gegenwart eines Verdünnungsmittels wie beispielsweise Ethanol, bei Temperaturen zwischen 0°C und 100°C cyclisiert, die aus dem Malonester der Formel (VIII) sich ergebenden 4-Ethoxycarbonylpyrazolin-5-one der Formel (IX)

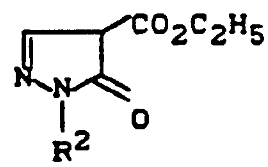

(IX)

in welcher

$R^2$ die oben angegebene Bedeutung hat,

in einem Zwischenschritt, nach üblichen Methoden, beispielsweise mit wäßriger Salzsäure bei Temperaturen zwischen 50°C und 120°C verseift und decarboxyliert, und die so erhältlichen Pyrazolin-5-one der Formel (X)

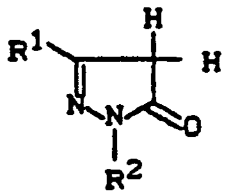

(X)

in welcher

$R^1$ und $R^2$ die oben angegebene Bedeutung haben,

in einer 2. Stufe (bzw. 3. Stufe) mit einem Nitrosierungsmittel, wie beispielsweise Isopentylnitrit oder Natriumnitrit, gegebenenfalls in Gegenwart eines Verdünnungsmittels, wie beispielsweise Ethanol, Wasser oder wäßrige Salzsäure und gegebenenfalls in Gegenwart einer Base, wie beispielsweise Natriummethylat, bei Temperaturen zwischen −20°C und +50°C umsetzt.

Die zur Durchführung des erfindungsgemäßen Verfahrens (a) als Ausgangsstoffe benötigten Alkylierungsmittel sind durch die Formel (III) allgemein definiert. In dieser Formel (III) steht Het für diejenigen Reste, die bereits im Zusammenhang mit der Beschreibung der erfindungsgemäßen Stoffe der Formel (I) für diese Substituenten genannt wurden. X' steht vorzugsweise für Halogen, insbesondere Chlor, Brom oder Iod, oder für gegebenenfalls substituiertes Alkylsulfonyloxy, Alkoxysulfonyloxy oder Arylsulfonyloxy wie beispielsweise Methansulfonyloxy, Methoxysulfonyloxy, Trifluormethansulfonyloxy oder p-Toluolsulfonyloxy.

Die Alkylierungsmittel der Formel (III) sind allgemein bekannte Verbindungen der organischen Chemie.

Die β-Ketoester der Formel (VII) bzw. der Ethoxymethylenmalonester der Formel (VIII) sind ebenfalls allgemein bekannt.

Die zur Durchführung des erfindungsgemäßen Verfahrens (b) als Ausgangsstoffe benötigten Alkoximinocarbonsäureester sind durch die Formel (IV) allgemein definiert. In dieser Formel (IV) stehen $R^1$ und Het für diejenigen Reste, die bereits im Zusammenhang mit der Beschreibung der erfindungsgemäßen Stoffe der Formel (I) für diese Substituenten genannt wurden. R steht vorzugsweise für geradkettiges oder verzweigtes Alkyl mit 1 bis 4 Kohlenstoffatomen, insbesondere für Methyl oder Ethyl.

Die Alkoximinocarbonsäureester der Formel (IV) sind noch nicht bekannt.

Man erhält sie, wenn man Hydroximinocarbonsäureester der Formel (XI),

```
     O  O
     ‖  ‖
R¹-C-C-C-OR          (XI)
       ‖
       N-OH
```

in welcher

R und $R^1$ die oben angegebene Bedeutung haben,

mit Alkylierungsmitteln der Formel (III),

$$\text{Het-CH}_2\text{-X}' \qquad \text{(III)}$$

in welcher

Het die oben angegebene Bedeutung hat und

X' für eine elektronenanziehende Abgangsgruppe, insbesondere für Chlor, Brom oder Iod, oder für gegebenenfalls substituirtes Alkylsulfonyloxy, Alkoxysulfonyloxy oder Arylsulfonyloxy wie beispielsweise Methansulfonyloxy, Methoxysulfonyloxy, Trifluormethansulfonyloxy oder p-Toluolsulfonyloxy steht,

gegebenenfalls in Gegenwart eines Verdünnungsmittels wie beispielsweise Acetonitril sowie gegebenenfalls in Gegenwart eines Säurebindemittels, wie beispielsweise Triethylamin, bei Temperaturen zwischen +10°C und +80°C umsetzt.

Die Hydroximinocarbonsäureester der Formel (XI) sind allgemein bekannte Verbindungen der organischen Chemie (vgl. z.B. Helv. Chim. Acta 67, 906-915 [1984]; Franz. Patentanmeldung Nr. 2 434 572; Yakugaku Zasshi 87, 1209-1211 [1967] oder Chem. Ber. 100, 1245-1247 [1967]).

Die zur Durchführung des erfindungsgemäßen Verfahrens (c) als Ausgangsstoffe benötigten 4-Alkoximinopyrazolin-5-one sind durch die Formel (Ia) allgemein definiert. In dieser Formel (Ia) stehen $R^1$ und Het vorzugsweise für diejenigen Reste, die bereits im Zusammenhang mit der Beschreibung der erfindungsgemäßen Stoffe der Formel (I) für diese Substituenten genannt wurden.

Die 4-Alkoximinopyrazolin-5-one der Formel (Ia) sind erfindungsgemäße Verbindungen und erhältlich mit Hilfe der erfindungsgemäßen Verfahren (a) oder (b).

Die zur Durchführung des erfindungsgemäßen Verfahrens (c) weiterhin als Ausgangsstoffe benötigten Alkylierungsmittel sind durch die Formel (VI) allgemein definiert. In dieser Formel (VI) steht $R^{2'}$ vorzugsweise für diejenigen Reste, die bereits im Zusammenhang mit der Beschreibung der erfindungsgemäßen Stoffe der Formel (I) für den Susbtituenten $R^2$ genannt wurden, mit Ausnahme des Wasserstoffrestes und der gegebenenfalls substituierten Arylreste. Y steht vorzugsweise für diejenigen Abgangsgruppen, die bereits bei der Beschreibung der Alkylierungsmittel der Formel (III) für den Substituenten X' genannt wurden.

Die Alkylierungsmittel der Formel (VI) sind ebenfalls allgemein bekannte Verbindungen der organischen Chemie.

Als Verdünnungsmittel zur Durchführung des erfindungsgemäßen Verfahrens (a) kommen inerte organische Lösungsmittel oder wäßrige Systeme in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder diethylether:

Ketone, wie Aceton oder Butanon; Nitrile, wie Acetonitril oder Propionitril; Amide, wie Dimethylformamid, Dimethylacetamid, N-Methylformanilid, N--Methylpyrrolidon oder Hexamethylphosphorsäuretriamid; Ester, wie Essigsäureethylester; Sulfoxide, wie Dimethylsulfoxid, oder auch Wasser oder wäßrig-organische Zweiphasen-Gemische, wie Dichlormethan-Wasser oder Toluol-Wasser.

Das erfindungsgemäße Verfahren (a) wird gegebenenfalls in Gegenwart eines Säurebindemittels durchgeführt. Als solche kommen alle üblichen anorganischen oder organischen Basen in Frage. Hierzu gehören beispielsweise Alkalimetallhydroxide, -amide, -alkoholate oder -hydride, wie Natriumhydroxid oder Kaliumhydroxid, Natriummethylat oder Kaliumt-butylat, Natriumhydrid oder Natriumamid; Alkalimetallcarbonate, wie Natriumcarbonat, Kaliumcarbonat oder Natriumhydrogencarbonat sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperatur kann bei der Durchführung des erfindungsgemäßen Verfahrens (a) in einem größeren Bereich variiert werden. Im allgemeinen arbeitet man bei Temperaturen zwischen −20°C und +200°C, vorzugsweise bei Temperaturen zwischen 0°C und +150°C.

Bei der Durchführung des erfindungsgemäßen Verfahrens (a) setzt man pro Mol 4-Oximino-pyrazolin-5-on der Formel (II) im allgemeinen 1,0 bis 3,0 Mol, vorzugsweise 1,0 bis 1,5 Mol, an Alkylierungsmittel der Formel (III) und gegebenenfalls 1,0 bis 3,0 Mol, vorzugsweise 1,0 bis 1,5 Mol, Säurebindemittel ein.

Führt man die Umsetzung in einem organischwäßrigen Zweiphasensystem durch, kann man gegebenenfalls in Gegenwart von 0,1 bis 1 Mol eines geeigneten Phasentransferkatalysators wie beispielsweise einer quartären Ammonium- oder Phosphoniumverbindung arbeiten. Beispielhaft seien Triethylbenzylammoniumchlorid und Benzyl-dodecyldimethylammoniumchlorid genannt.

Die Aufarbeitung und Isolierung der Reaktionsprodukte der Formel (I) erfolgt nach üblichen Methoden.

Als Verdünnungsmittel zur Durchführung des erfindungsgemäßen Verfahrens (b) kommen ebenfalls inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder-diethylether oder Alkohole, wie Methanol, Ethanol oder Propanol.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 150°C, vorzugsweise bei Temperaturen zwischen 20°C und 120°C.

Zur Durchführung des erfindungsgemäßen Verfahrens (b) setzt man pro Mol Alkoximinocarbonsäureester der Formel (IV) im allgemeinen 0,8 bis 2,5 Mol, vorzugsweise 1,0 bis 1,2 Mol, an Hydrazin-Derivat der Formel (V) ein. Die Reaktionsdurchführung, Aufarbeitung und Isolierung der Reaktionsprodukte der Formel (I) erfolgt nach üblichen Methoden.

Als Verdünnungsmittel zur Durchführung des erfindungsgemäßen Verfahrens (c) kommen ebenfalls inerte organische Lösungsmittel oder wäßrige Systeme in Frage.

Vorzugsweise verwendet man die bei Verfahren (a) angegebenen organischen Lösungsmittel oder wäßrig-organischen Zweiphasen-Gemische.

Das erfindungsgemäße Verfahren (c) wird gegebenenfalls in Gegenwart eines Säurebindemittels durchgeführt. Vorzugsweise verwendet man als solche die bei Verfahren (a) angegebenen anorganischen oder organischen Basen.

Die Reaktionstemperaturen können bei der Durchführung des erfindungsgemäßen Verfahrens (c) ebenfalls in einem größeren Bereich variiert werden. Im allgemeinen arbeitet man bei Temperaturen zwischen −20°C und +200°C, vorzugsweise bei Temperaturen zwischen 0°C und +150°C.

Bei der Durchführung des erfindungsgemäßen Verfahrens (c) setzt man pro Mol 4-Alkoximino-pyrazolin-5-on der Formel (Ia) im allgemeinen 1,0 bis 3,0 Mol, vorzugsweise 1,0 bis 1,5 Mol, Alkylierungsmittel der Formel (VI) und gegebenenfalls 0,5 bis 3,0 Mol, vorzugsweise 0,6 bis 1,5 Mol, Säurebindemittel ein.

Die Reaktionsdurchführung, Aufarbeitung und Isolierung der Reaktionsprodukte der Formel (I) erfolgt, wie bei Verfahren (a) beschrieben bzw. nach allgemein üblichen Verfahren.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten verursacht beispielsweise durch Cochliobolus sativus, Leptosphaeria nodorum, Erysiphe graminis oder Pyrenophora teres, zur Bekämpfung von Gemüsekrankheiten verursacht beispielsweise durch den Erreger der Tomatenbraunfäule (Phytophthora infestans) oder zur Bekämpfung von Reiskrankheiten wie beispielsweise gegen den Erreger der Reisfleckenkrankheit (Pyricularia oryzae) oder gegen den Erreger Pellicularia sasakii, eingesetzt werden. Dabei zeigen die erfindungsgemäßen Wirkstoffe neben protektiver Wirksamkeit auch systemische Eigenschaften und darüberhinaus auch eine gute Wirksamkeit gegen die Bakterienkrankheit Erwinia amylovora (Feuerbrand). Die fungizide Wirksamkeit der erfindungsgemäßen Verbindungen zeigt sich auch in vitro im Agarplattentest.

Die erfindungsgemäßen Wirkstoffe zeigen neben einer hervorragenden protektiven Wirsamkeit auch sehr gute systemische Eigenschaften. Sie zeichnen sich durch breite fungizide in-vitro-Wirkung und durch zusätzliche bakterizide Eigenschaften aus.

Die Wirkstoffe können in den üblichen Formulierungen überführt werden wie Lösungen, Emulsionen, Suspensionen, Pulver, Stäubemittel, Schäume, Pasten, lösliche Pulver, Granulate, Aerosole, Suspension-Emulsionskonzentrate, Saatgutpuder, Wirkstoff-imprägnierte Natur- und synthetische Stoffe, Feinstverkapselungen in polymeren Stoffen und Hüllmassen für Saatgut, ferner in Formulierungen mit Brennsätzen wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe wie Cyclohexan oder Paraffine, z.B. Erdölfraktionen, Alkohole wie Butanol oder Glykol sowie deren Ether und Ester, Ketone wie Aceton, Methylethylketon, Methylisobutylketon oder Cyclohexanon, stark polare Lösungsmittel wie Dimethylformamid und Dimethylsulfoxid sowie Wasser. Mit verflüssigten gasförmigen Streckmitteln oder deren Trägerstoffen sind solche Flüssigkeiten gemeint, welche bei normaler Temperatur und unter Normaldruck gasförmig sind, z.B. Aerosol-Treibgase wie Halogenkohlenwasserstoffe sowie Butan, Propan, Stickstoff und Kohlendioxid. Als feste Trägerstoffe kommen in Frage: z.B. natürliche Gesteinsmehle wie Kaoline, Tonerden, Talkum, Kreide, Quarz, Attapulgit, Montmorillonit oder Diatomenerde und synthetische Gesteinsmehle wie hochdisperse Kieselsäure, Aluminiumoxid und Silikate. Als feste Trägerstoffe kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden .

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatzgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02% am Wirkungsort erforderlich.

Herstellungsbeispiele:

*Beispiel 1*

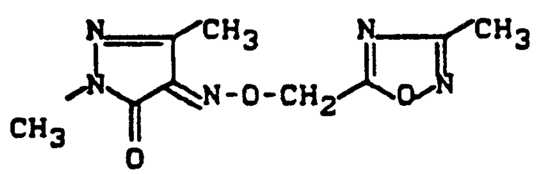

(Verfahren a)

Zu 12 g (0,085 Mol) 1,3-Dimethyl-4-hydroximino-pyrazolin-5-on in 100 ml Acetonitril gibt man unter Rühren zunächst 11,1 g (0,11 Mol) Triethylamin und anschließend tropfenweise 11,35 g (0,085 Mol) 3-Methyl-5-chlormethyl-1,2,4-oxadiazol. Nach beendeter Zugabe erwärmt man für eine Stunde auf 50°C, entfernt das Lösungsmittel im Vakuum, nimmt den Rückstand in Chloroform auf, wäscht mehrmals mit Wasser, trocknet über Magnesiumsulfat und entfernt das Lösungsmittel im Vakuum. Das zurückbleibende Öl wird chromatographisch (Kieselgel/Laufmittel: Chloroform/Ether 10:1) gereinigt und aus Petrolether kristallisiert.

Man erhält 9,9 g (49% der Theorie) an 4-[(3-Methyl-1,2,4-oxadiazol-5-yl)-methoximino]-1,3-dimethyl-pyrazolin-5-on vom Schmelzpunkt 54°C.

Herstellung der Ausgangsverbindung

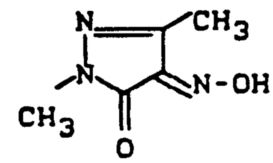

Zu 175 g (1,56 Mol) 1,3-Dimethyl-pyrazolin-5-on und 84,4 g (1,56 Mol) Natriummethylat in 1 l absolutem Ethanol gibt man unter Rühren und Eiskühlung tropfenweise 183 g (1,56 Mol) Isopentylnitrit, so daß die Innentemperatur 25°C bis 30°C nicht übersteigt. Nach beendeter Zugabe rührt man weitere 24 Stunden bei Raumtemperatur und saugt das ausgefallene Natriumsalz des 1,3-Dimethyl-4-hydroximino-pyrazolin-5-ons ab. Das kristalline Produkt wird in 1 l Wasser gelöst und mit Eisessig angesäuert. Zur vollständigen Fällung kühlt man für mehrere Stunden auf 0°C und saugt dann das Produkt ab.

Man erhält 162 g (74% der Theorie) an 1,3-Dimethyl-4-hydroximino-pyrazolin-5-on vom Schmelzpunkt 93°C.

*Beispiel 2*

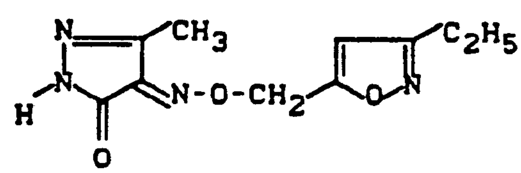

(Verfahren b)

320 g (1,194 Mol) β-Keto-α-[(3-ethylisoxazol-5-yl)-methoximino]-buttersäureethylester in 400 ml Ethanol werden bei Raumtemperatur tropfenweise unter Rühren mit 59,5 g (1,19 Mol) Hydrazinhydrat auf 70°C erwärmt. Nach beendeter Zugabe rührt man weitere 3 Stunden bei Rückflußtemperatur und destilliert dann das Lösungsmittel aus der abgekühlten Reaktionsmischung unter vermindertem Druck ab. Der Rückstand, der beim Verrühren mit Ethanol/Diethylether kristallisiert, wird abgesaugt und getrocknet.

Man erhält 205 g (73% der Theorie) an 4-[(3-Ethylisoxazol-5-yl)-methoximino]-3-methyl-pyrazolin-5-on vom Schmelzpunkt 118°C.

*Beispiel 3*

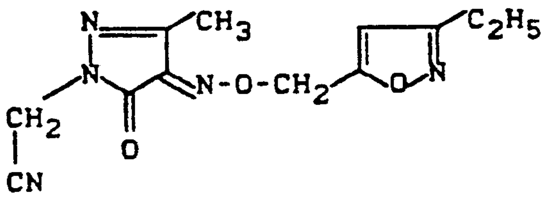

(Verfahren c)

205 g (0,869 Mol) 4-[3-Ethylisoxazol-5-yl)-methoximino]-3-methyl-pyrazolin-5-on, 82,8 g (0,6 Mol) gepulvertes Kaliumcarbonat und 63,4 ml (1 Mol) Chloracetonitril in 1000 ml Aceton werden 4 Stunden unter Rückfluß erhitzt. Danach gibt man weitere 21,1 ml (0,33 Mol) Chloracetonitril zu und erhitzt für weitere 2 Stunden unter Rückfluß. Die abgekühlte Reaktionsmischung wird im Vakuum eingeengt, der Rückstand in Dichlormethan aufgenommen, die unlöslichen Bestandteile abfiltriert, das Filtrat mehrfach gewaschen, über Natriumsulfat getrocknet, im Vakuum eingeengt und chromatographisch (Kieselgel) gereinigt.

Man erhält 74,3 g (35% der Theorie) an 1-Cyanmethyl-4-](3-ethylisoxazol-5-yl)-methoximino]-3--methyl-pyrazolin-5-on als Öl.

$^1$H-NMR ($CDCl_3$/Tetramethylsilan als innerer Standard): δ = 5,5 ppm (=N-O-$CH_2$-)

In entsprechender Weise und gemäß den allgemeinen Angabe zur Herstellung erhält man die folgenden substituierten Pyrazolin-5-one der allgemeinen Formel (I):

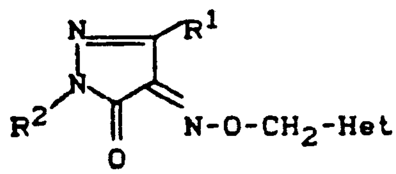

(I)

| Beispiel Nr. | $R^1$ | $R^2$ | Het | Physikalische Daten |
|---|---|---|---|---|
| 4 | H | $CH_3$ | 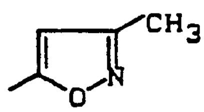 | Fp 146-148°C |
| 5 | $CH_3$ | H | 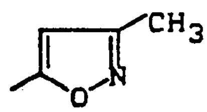 | Fp 135°C |
| 6 | $CH_3$ | $CH_3$ | 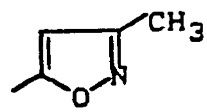 | Fp 101°C |
| 7 | $CH_3$ | $(CH_3)_3C$- | 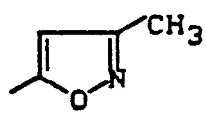 | $^1$H-NMR*) 5,41 ppm |
| 8 | $CH_3$ | $CH_2$=CH-$CH_2$- | 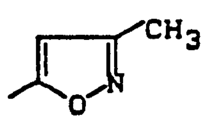 | $^1$H-NMR*) 5,45 ppm |
| 9 | $CH_3$ | NC-$CH_2$- | 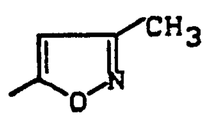 | $^1$H-NMR*) 5,5 ppm |
| 10 | $CH_3$ | HO-$CH_2$-$CH_2$- | 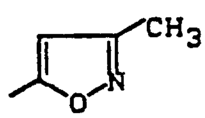 | $^1$H-NMR*) 5,5 ppm |

| Beispiel Nr. | $R^1$ | $R^2$ | Het | Physikalische Daten |
|---|---|---|---|---|
| 11 | $CH_3$ | $CH_3O$-CO-$CH_2$- | 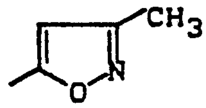 | $^1$H-NMR*) 5,6 ppm |
| 12 | $CH_3$ | 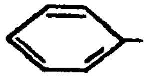 | 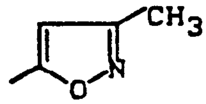 | $^1$H-NMR*) 5,0 ppm |
| 13 | $CH_3$ | 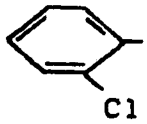 | 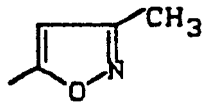 | $^1$H-NMR*) 5,5 ppm |
| 14 | $CH_3$ | 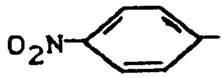 | 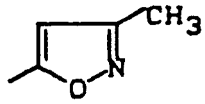 | Fp 180°C (Zers.) |
| 15 | $CH_3$ | 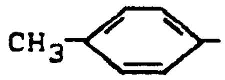 | 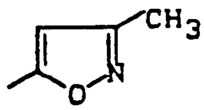 | Fp 104°C |
| 16 | $C_2H_5$ | $CH_3$ | 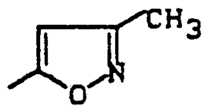 | $^1$H-NMR*) 5,5 ppm |
| 17 | $CH_3$-$(CH_2)_2$- | $CH_3$ | 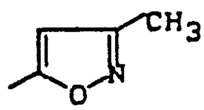 | $^1$H-NMR*) 5,46 ppm |
| 18 | $(CH_3)_3C$- | $CH_3$ | 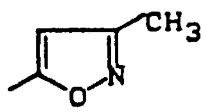 | Fp 73°C |
| 19 | $C_2H_5$-O-C(=O)- | $CH_3$ | 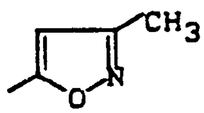 | Fp 100°C |
| 20 | 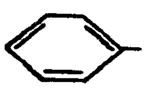 | $CH_3$ | 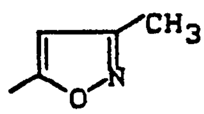 | Fp 83°C |
| 21 | $CH_3$ | $CH_3$ | 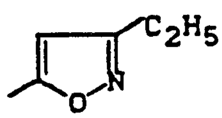 | $^1$H-NMR*) 5,5 ppm |
| 22 | $CH_3$ | $(CH_3)_3C$- | 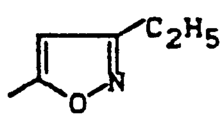 | $^1$H-NMR*) 5,4 ppm |

| Beispiel Nr | $R^1$ | $R^2$ | Het | Physikalische Daten |
|---|---|---|---|---|
| 23 | $CH_3$ | $HO-CH_2-CH_2-$ | 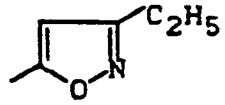 | $^1$H-NMR*) 5,5 ppm |
| 24 | $CH_3$ | 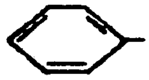 | 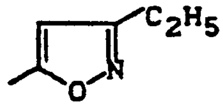 | $^1$H-NMR*) 5,0 ppm |
| 25 | $CH_3$ | 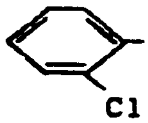 | 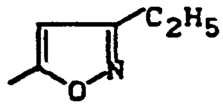 | $^1$H-NMR*) 5,52 ppm |
| 26 | $CH_3$ | 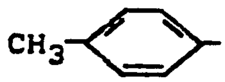 | 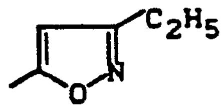 | Fp 103°C |
| 27 | $C_2H_5$ | $CH_3$ | 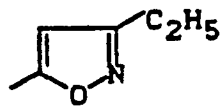 | $^1$H-NMR*) 5,58 ppm |
| 28 | $CH_3-(CH)_2-$ | $CH_3$ | 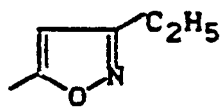 | $^1$H-NMR*) 5,46 ppm |
| 29 | $(CH_3)_3C-$ | $CH_3$ | 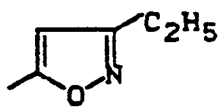 | Fp 61°C |
| 30 | $C_2H_5-O-\overset{O}{\overset{\Vert}{C}}-$ | $CH_3$ | 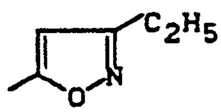 | Fp 88°C |
| 31 | 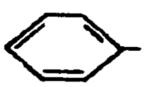 | $CH_3$ | 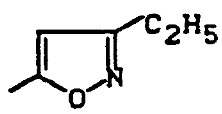 | Fp 78°C |
| 32 | H | $CH_3$ | 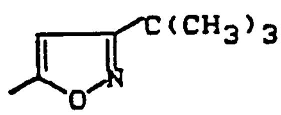 | $^1$H-NMR*) 5,45 ppm |
| 33 | $CH_3$ | $CH_3$ | 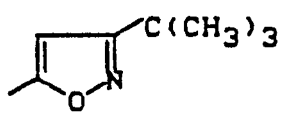 | $^1$H-NMR*) 5,5 ppm |
| 34 | $CH_3$ | $NC-CH_2-CH_2-$ | 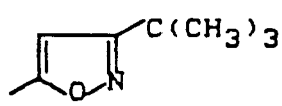 | $^1$H-NMR*) 5,5 ppm |

| Beispiel Nr. | $R^1$ | $R^2$ | Het | Physikalische Daten |
|---|---|---|---|---|
| 35 | $CH_3$ | $HO-CH_2-CH_2-$ | 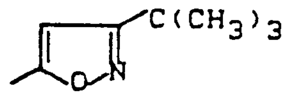 | $^1$H-NMR*) 5,5 ppm |
| 36 | H | H | 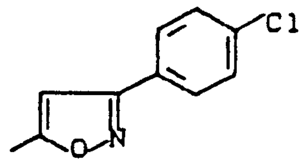 | Fp 124-126 |
| 37 | H | $CH_3$ | 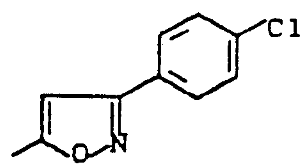 | $^1$H-NMR*) 5,5 ppm |
| 38 | $CH_3$ | H | 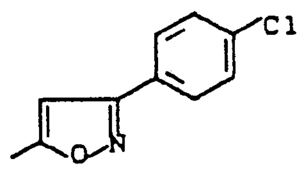 | Fp 163°C |
| 39 | $CH_3$ | $CH_3$ | 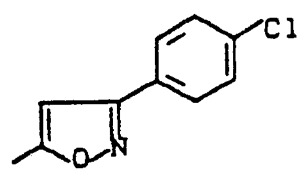 | $^1$H-NMR*) 5,5 ppm |
| 40 | $CH_3$ | $NC-CH_2-$ | 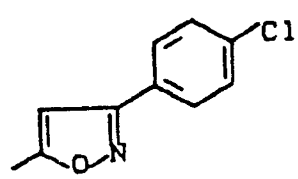 | $^1$H-NMR*) 5,56 ppm |
| 41 | $CH_3$ | $NC-CH_2-CH_2-$ | 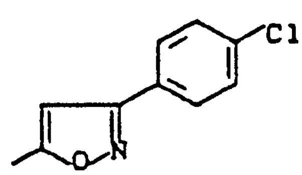 | $^1$H-NMR*) 5,5 ppm |

| Beispiel Nr. | $R^1$ | $R^2$ | Het | Physikalische Daten |
|---|---|---|---|---|
| 42 | $CH_3$ | $C_2H_5O-\overset{\overset{O}{\|}}{C}-CH_2-$ | 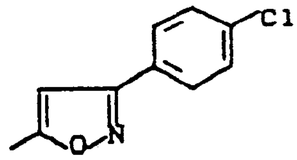 | Fp 108°C |
| 43 | $C_2H_5$ | $CH_3$ | 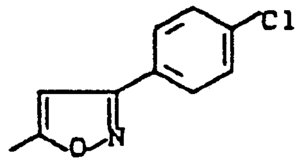 | $^1$H-NMR*) 5,5 ppm |
| 44 | $CH_3O-CH_2-$ | $NC-CH_2-CH_2-$ | 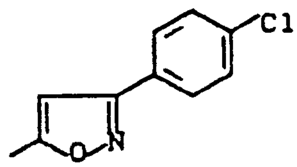 | $^1$H-NMR*) 5,6 ppm |
| 45 | $CH_3O-\overset{\overset{O}{\|}}{C}-CH_2-$ | $CH_3$ | 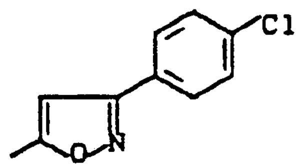 | $^1$H-NMR*) 5,53 ppm |
| 46 | $CH_3$ | $CH_3$ | 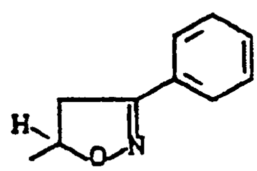 | $^1$H-NMR*) 5,28 ppm |
| 47 | $CH_3$ | $CH_3$ | 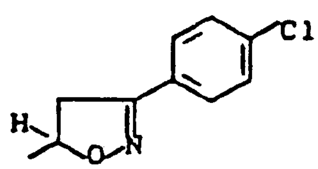 | Fp 135-140°C |
| 48 | $C_2H_5$ | $CH_3$ | 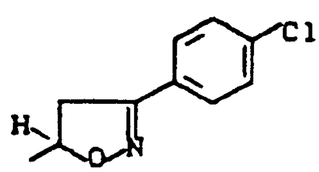 | Fp 133-140°C |

| Beispiel Nr. | $R^1$ | $R^2$ | Het | Physikalische Daten |
|---|---|---|---|---|
| 49 | $CH_3O-CH_2-$ | $CH_3$ | 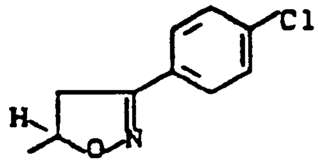 | $^1$H-NMR*) 4,6 ppm |
| 50 | $CH_3O-\overset{O}{\overset{\|}{C}}-CH_2-$ | $CH_3$ | 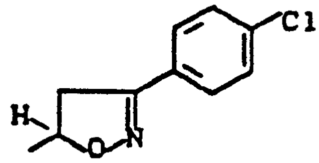 | $^1$H-NMR*) 5,52 ppm |
| 51 | H | $HO-CH_2-CH_2-$ | 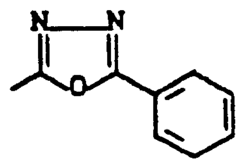 | Fp 118-119°C |
| 52 | $CH_3$ | $CH_3$ | 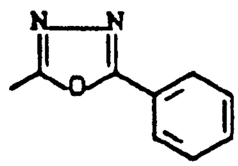 | $^1$H-NMR*) 5,66 ppm |
| 53 | $CH_3$ | $NC-CH_2-CH_2-$ | 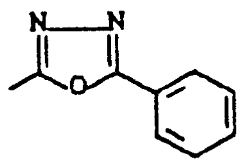 | $^1$H-NMR*) 5,66 ppm |
| 54 | $C_2H_5$ | $CH_3$ | 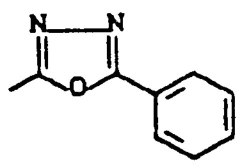 | Fp 82°C |
| 55 | $CH_3O-CH_2-$ | $CH_3$ | 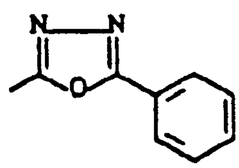 | $^1$H-NMR*) 5,66 ppm |
| 56 | $CH_3O-CH_2-$ | $NC-CH_2-CH_2-$ | 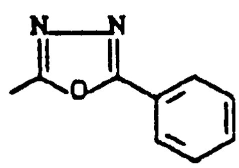 | Fp 106°C |

| Beispiel Nr. | $R^1$ | $R^2$ | Het | Physikalische Daten |
|---|---|---|---|---|
| 57 | $CH_3$ | $(CH_3)_3C$- | 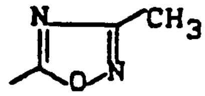 | $^1$N-NMR*) 5,58 ppm |
| 58 | $CH_3$ | 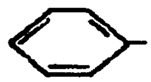 | 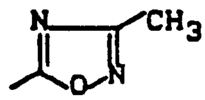 | Fp 98°C |
| 59 | $CH_3$ | 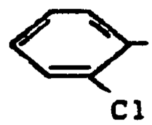 | 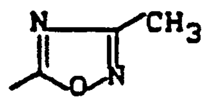 | $^1$H-NMR*) 5,66 ppm |
| 60 | $CH_3$ | 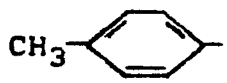 | 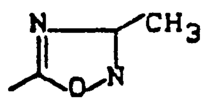 | Fp 98°C |
| 61 | $CH_3$ | 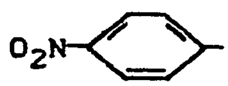 | 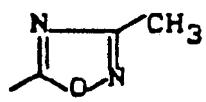 | Fp 160°C |
| 62 | $C_2H_5$ | $CH_3$ | 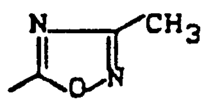 | $^1$H-NMR*) 5,6 ppm |
| 63 | 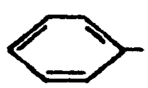 | $CH_3$ | 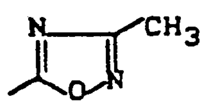 | Fp 75°C |
| 64 | $CH_3$-$(CH_2)_2$- | $CH_3$ | 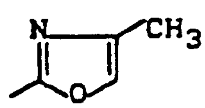 | $^1$H-NMR*) 5,6 ppm |
| 65 | $(CH_3)_3C$- | $CH_3$ | 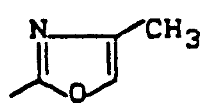 | $^1$H-NMR*) 5,58 |
| 66 | H | $CH_3$ | 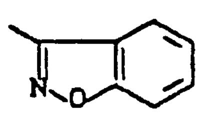 | $^1$H-NMR*) 5,83 ppm |

| Beispiel Nr. | $R^1$ | $R^2$ | Het | Physikalische Daten |
|---|---|---|---|---|
| 67 | H | $NC-CH_2-CH_2-$ | 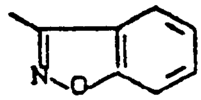 | $^1H$-NMR*) 5,83 ppm |
| 68 | H | $HO-CH_2-CH_2-$ | 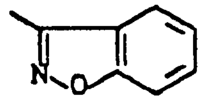 | $^1H$-NMR*) 5,83 ppm |
| 69 | $CH_3$ | H | 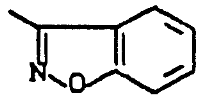 | Fp 102°C |
| 70 | $CH_3$ | $CH_3$ | 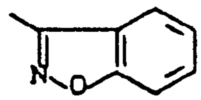 | $^1H$-NMR*) 5,83 ppm |
| 71 | $CH_3$ | $NC-CH_2-CH_2-$ | 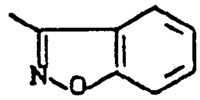 | $^1H$-NMR*) 5,83 ppm |
| 72 | $CH_3$ | $HO-CH_2-CH_2-$ | 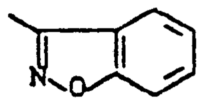 | Fp 114-116°C |
| 73 | $CH_3O-CH_2-$ | $NC-CH_2-CH_2-$ | 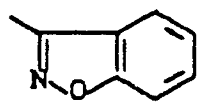 | $^1H$-NMR*) 5,91 ppm |
| 74 | H | H | 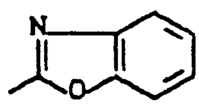 | Fp 134°C |
| 75 | H | $CH_3$ | 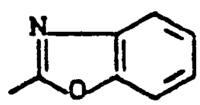 | $^1H$-NMR*) 5,66 ppm |
| 76 | $CH_3$ | H | 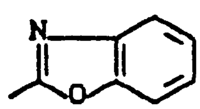 | Fp 130-132°C |
| 77 | $CH_3$ | $CH_3$ | 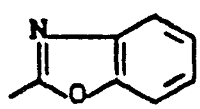 | Fp 110-114°C |
| 78 | $CH_3$ | $NC-CH_2-$ | 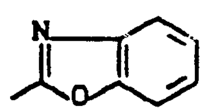 | $^1H$-NMR*) 5,66 ppm |

| Beispiel Nr. | $R^1$ | $R^2$ | Het | Physikalische Daten |
|---|---|---|---|---|
| 79 | $CH_3$ | $NC-CH_2-CH_2$ | 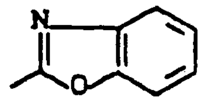 | $^1$H-NMR*) 5,66 ppm |
| 80 | $CH_3$ | $HO-CH_2-CH_2-$ | 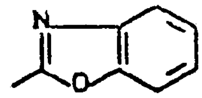 | $^1$H-NMR*) 5,66 ppm |
| 81 | H | H | 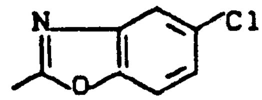 | Fp 112-115°C |
| 82 | H | $CH_3$ | 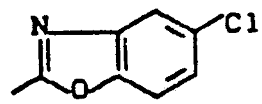 | Fp 115°C |
| 83 | $CH_3$ | H | 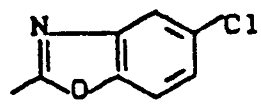 | Fp 160°C |
| 84 | $CH_3$ | $CH_3$ | 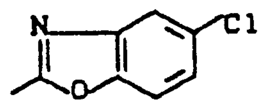 | $^1$H-NMR*) 5,66 ppm |
| 85 | $CH_3$ | $NC-CH_2-$ | 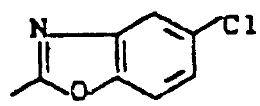 | $^1$H-NMR*) 5,7 ppm |
| 86 | $CH_3$ | $NC-CH_2-CH_2-$ | 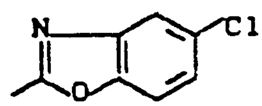 | $^1$H-NMR*) 5,66 ppm |
| 87 | $CH_3$ | $HO-CH_2-CH_2-$ | 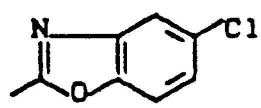 | Fp 146-147°C |
| 88 | $CH_3$ | H | 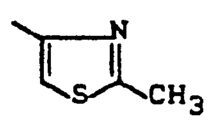 | Fp 104°C |
| 89 | $CH_3$ | $CH_3$ | 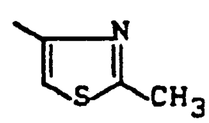 | $^1$H-NMR*) 5,5 ppm |
| 90 | $CH_3$ | $(CH_3)_3C-$ | 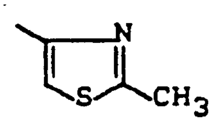 | $^1$H-NMR*) 5,5 ppm |

| Beispiel Nr. | $R^1$ | $R^2$ | Het | Physikalische Daten |
|---|---|---|---|---|
| 91 | $CH_3$ | $NC-CH_2-$ | 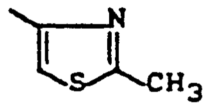 | Fp 81°C |
| 92 | $CH_3$ | $NC-CH_2-CH_2-$ | 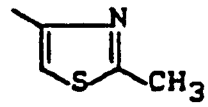 | Fp 82°C |
| 93 | $CH_3$ | $HO-CH_2-CH_2-$ | 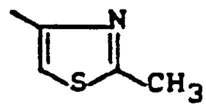 | $^1$H-NMR*) 5,5 ppm |
| 94 | $CH_3$ | 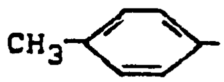 | 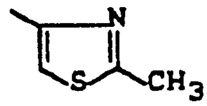 | $^1$H-NMR*) 5,5 ppm |
| 95 | $C_2H_5$ | $CH_3$ | 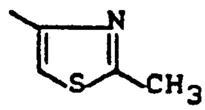 | $^1$H-NMR*) 5,53 ppm |
| 96 | $CH_3O-CH_2-$ | $CH_3$ | 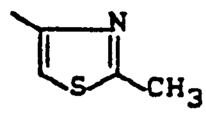 | $^1$H-NMR*) 5,53 ppm |
| 97 | $CH_3O-CH_2-$ | $NC-CH_2-CH_2-$ | 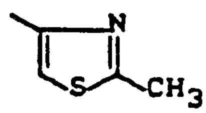 | $^1$H-NMR*) 5,53 ppm |
| 98 | $C_2H_5O-\overset{O}{\overset{\Vert}{C}}-$ | $CH_3$ | 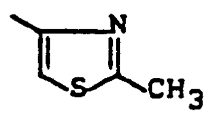 | Fp 119°C |
| 99 | H | $NC-CH_2-CH_2-$ | 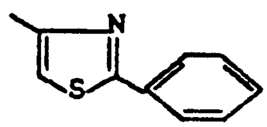 | Fp 116°C |
| 100 | $CH_3$ | H | 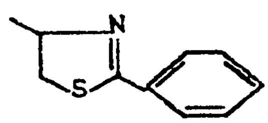 | $^1$H-NMR*) 5,63 ppm |
| 101 | $CH_3$ | $CH_3$ | 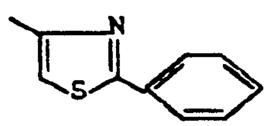 | Fp 91°C |

| Beispiel Nr. | $R^1$ | $R^2$ | Het | Physikalische Daten |
|---|---|---|---|---|
| 102 | $CH_3$ | $NC-CH_2-$ | 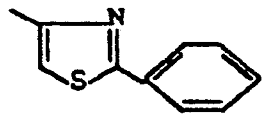 | Fp 130°C |
| 103 | $CH_3$ | $NC-CH_2-CH_2-$ | 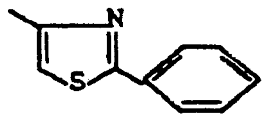 | Fp 97°C |
| 104 | $CH_3$ | $HO-CH_2-CH_2-$ | 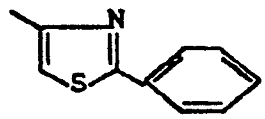 | Fp 94°C |
| 105 | $CH_3$ | 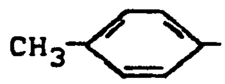 | 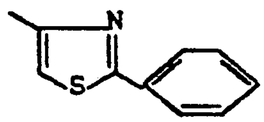 | $^1$H-NMR*) 5,65 ppm |
| 106 | $CH_3$ | 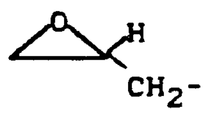 | 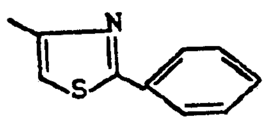 | $^1$H-NMR*) 5,6 ppm |
| 107 | $CH_3O-CH_2-$ | $CH_3$ | 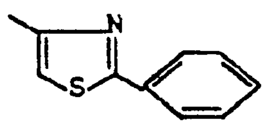 | $^1$H-NMR*) 5,66 ppm |
| 108 | $CH_3O-CH_2-$ | $NC-CH_2-CH_2-$ | 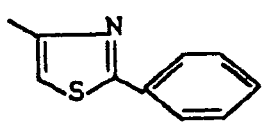 | $^1$H-NMR*) 5,66 ppm |
| 109 | $C_2H_5-O-\overset{O}{\overset{\parallel}{C}}-$ | $CH_3$ | 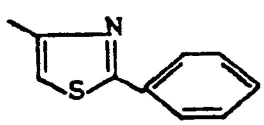 | Fp 95°C |
| 110 | H | $CH_3$ | 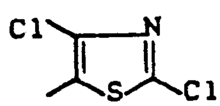 | $^1$H-NMR*) 5,5 ppm |
| 111 | $CH_3$ | H | 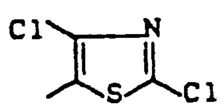 | Fp 154-156°C |

| Beispiel Nr. | $R^1$ | $R^2$ | Het | Physikalische Daten |
|---|---|---|---|---|
| 112 | $CH_3$ | $CH_3$ | 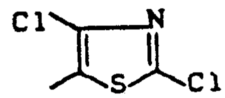 | Fp 108-110°C |
| 113 | $CH_3$ | $NC\text{-}CH_2\text{-}CH_2\text{-}$ | 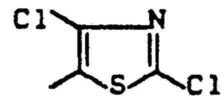 | $^1$H-NMR*) 5,5 ppm |
| 114 | $CH_3$ | $HO\text{-}CH_2\text{-}CH_2\text{-}$ | 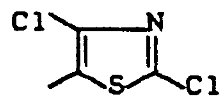 | Fp 91°C |
| 115 | H | $CH_3$ | 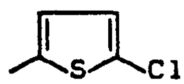 | $^1$H-NMR*) 5,5 ppm |
| 116 | $CH_3$ | H | 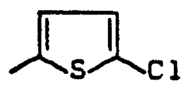 | Fp 116°C |
| 117 | $CH_3$ | $CH_3$ | 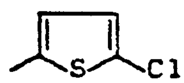 | $^1$H-NMR*) 5,5 ppm |
| 118 | $CH_3$ | $NC\text{-}CH_2\text{-}CH_2\text{-}$ | 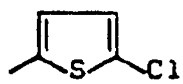 | $^1$-H-NMR*) 5,5 ppm |
| 119 | $CH_3$ | $HO\text{-}CH_2\text{-}CH_2\text{-}$ | 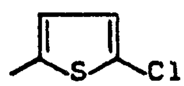 | $^1$H-NMR*) 5,5 ppm |
| 120 | H | H | 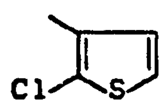 | Fp 80°C |
| 121 | H | $CH_3$ | 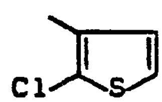 | $^1$H-NMR*) 5,43 ppm |
| 122 | H | $NC\text{-}CH_2\text{-}CH_2\text{-}$ | 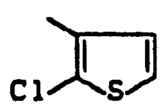 | $^1$H-NMR*) 5,5 ppm |
| 123 | $CH_3$ | H | 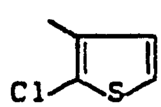 | Fp 120°C |

| Beispiel Nr. | $R^1$ | $R^2$ | Het | Physikalische Daten |
|---|---|---|---|---|
| 124 | $CH_3$ | $CH_3$ | 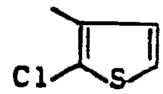 | Fp 64-66°C |
| 125 | $CH_3$ | $NC-CH_2-$ | 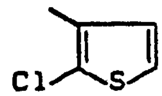 | $^{1}$H-NMR*) 5,5 ppm |
| 126 | $CH_3$ | $NC-CH_2-CH_2-$ | 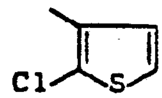 | $^{1}$H-NMR*) 5,43 ppm |
| 127 | $CH_3$ | $HO-CH_2-CH_2-$ | 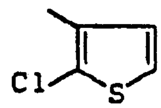 | Fp 94-96°C |
| 128 | H | $CH_3$ | 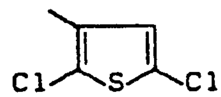 | $^{1}$H-NMR*) 5,5 ppm |
| 129 | $CH_3$ | H | 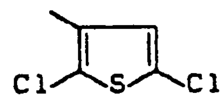 | Fp 120-122°C |
| 130 | $CH_3$ | $CH_3$ | 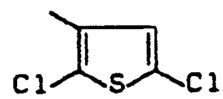 | Fp 65°C |
| 131 | $CH_3$ | $NC-CH_2-$ | 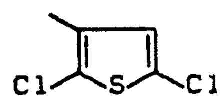 | $^{1}$H-NMR*) 5,5 ppm |
| 132 | $CH_3$ | $CH_3$ | 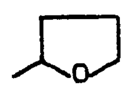 | $^{1}$H-NMR*) 4,55 ppm |
| 133 | $CH_3$ | H | 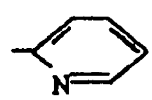 | Fp $>$ 150°C (Zers.) |
| 134 | $CH_3$ | $CH_3$ | 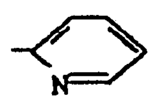 | Fp 80-82°C |
| 135 | $CH_3$ | $CH_3O-CO-CH_2-$ | 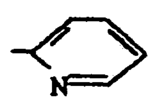 | $^{1}$H-NMR*) 5,6 ppm |

| Beispiel Nr. | $R^1$ | $R^2$ | Het | Physikalische Daten |
|---|---|---|---|---|
| 136 | $CH_3$ | $CH_3O-CO-CH_2-$ | 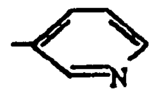 | $^1$H-NMR*) 5,6 ppm |
| 137 | $CH_3$ | $CH_3$ | 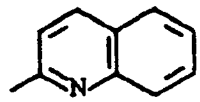 | $^1$H-NMR*) 5,8 ppm |
| 138 | $CH_3$ | $CH_3$ | 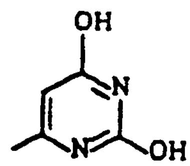 | Fp > 150°C (Zers.) |
| 139 | $CH_3$ | H | 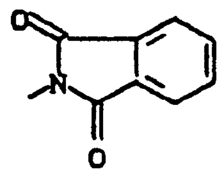 | Fp 160-165°C |
| 140 | $CH_3$ | $CH_3$ | 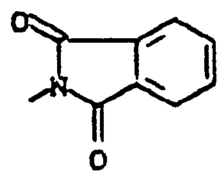 | Fp 168°C |
| 141 | $CH_3$ | $NC-CH_2-CH_2-$ | 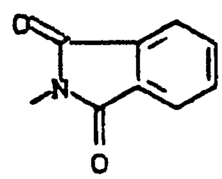 | Fp 150°C |
| 142 | $CH_3$ | 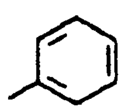 | 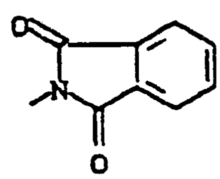 | Fp 140-145°C |
| 143 | $CH_3$ | 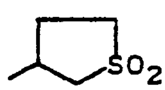 | 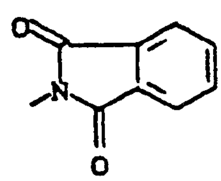 | Fp 182°C |
| 144 | $C_2H_5$ | $CH_3$ | 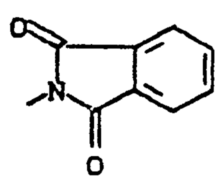 | Fp 177°C |

| Beispiel Nr. | $R^1$ | $R^2$ | Het | Physikalische Daten |
|---|---|---|---|---|
| 145 | $CH_3O-CH_2-$ | $CH_3$ | 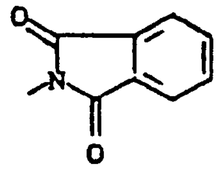 | Fp 165-175°C |
| 146 | $C_2H_5O-CH_2$ | $CH_3$ | 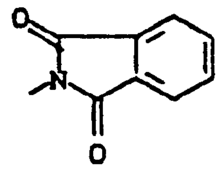 | Fp 138-145°C |
| 147 | $CH_3S-CH_2-$ | $CH_3$ | 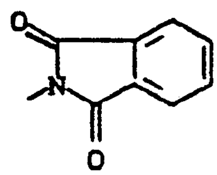 | $^1$H-NMR*) 5,95 ppm |
| 148 | $CH_3O-\overset{\overset{O}{\|}}{C}-CH_2-$ | $CH_3$ | 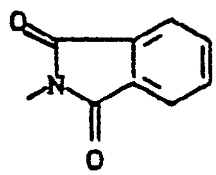 | Fp 111°C |
| 149 | H | H | 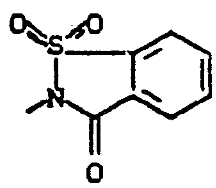 | Fp 184-186°C |
| 150 | H | $CH_3$ | 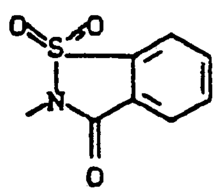 | Fp 90°C |
| 151 | H | $NC-CH_2-CH_2-$ | 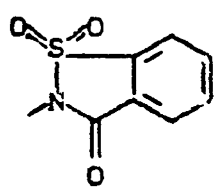 | Fp 172-176°C |
| 152 | $CH_3$ | H | 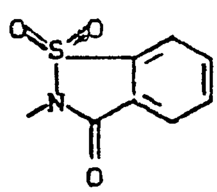 | Fp 206°C |

| Beispiel Nr. | $R^1$ | $R^2$ | Het | Physikalische Daten |
|---|---|---|---|---|
| 153 | $CH_3$ | $CH_3$ | 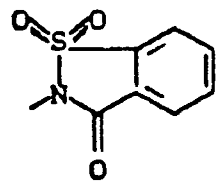 | Fp 177-179°C |
| 154 | $CH_3$ | $(CH_3)_3C$- | 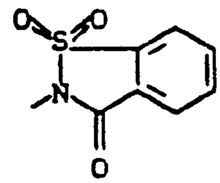 | $^1$H-NMR*) 6,0 ppm |
| 155 | $CH_3$ | NC-$CH_2$-$CH_2$- | 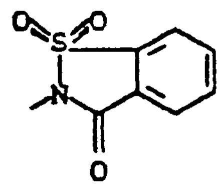 | Fp 176°C |
| 156 | $CH_3$ | HO-$CH_2$-$CH_2$- | 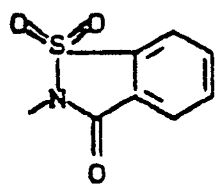 | $^1$H-NMR*) 6,0 ppm |
| 157 | $CH_3$ | 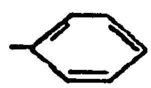 | 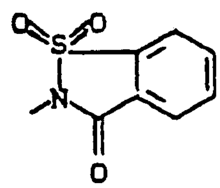 | $^1$H-NMR*) 6,05 ppm |
| 158 | $CH_3$ | 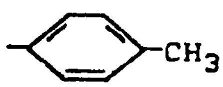 | 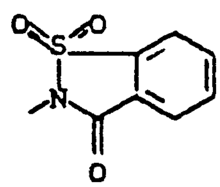 | Fp 210°C |
| 159 | $CH_3$ | 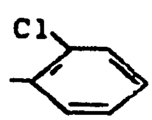 | 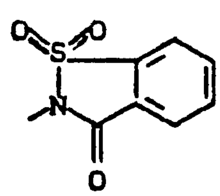 | Fp 148°C |
| 160 | $C_2H_5$ | H | 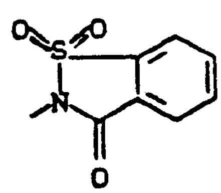 | Fp 173°C |

| Beispiel Nr. | $R^1$ | $R^2$ | Het | Physikalische Daten |
|---|---|---|---|---|
| 161 | $C_2H_5$ | $CH_3$ | 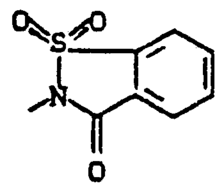 | Fp 142-144°C |
| 162 | $CH_3-(CH_2)_2-$ | H | 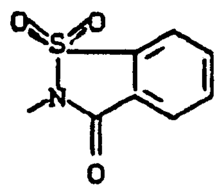 | Fp 100°C |
| 163 | $CH_3-(CH_2)_3-$ | H | 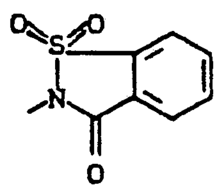 | Fp 110°C |
| 164 | $CH_3-(CH_2)_3-$ | $CH_3$ | 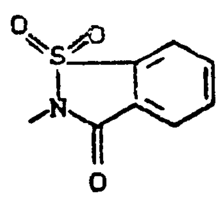 | Fp 105°C |
| 165 | $(CH_3)_3C-$ | $CH_3$ | 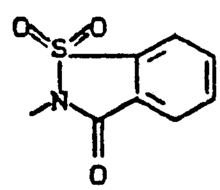 | Fp 190°C |
| 166 | $CH_3O-CH_2-$ | $CH_3$ | 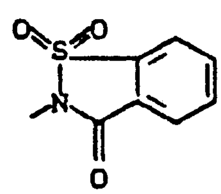 | Fp 128°C |
| 167 | $CH_3O-CH_2-$ | $NC-CH_2-CH_2-$ | 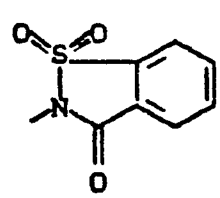 | $^1$H-NMR*) 6,12 ppm |
| 168 | $C_2H_5-O-\overset{O}{\overset{\|}{C}}-$ | $CH_3$ | 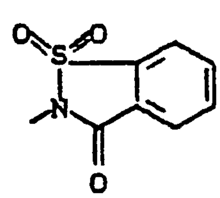 | $^1$H-NMR*) 6,06 ppm |

| Beispiel Nr. | $R^1$ | $R^2$ | Het | Physikalische Daten |
|---|---|---|---|---|
| 169 | 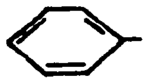 | $CH_3$ | 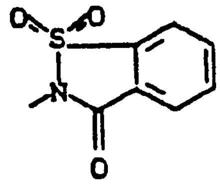 | Fp 196-198°C |
| 170 | $CH_3$ | 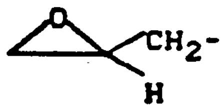 | 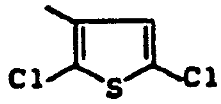 | $^1$H-NMR*) 5,4 ppm |
| 171 | $CH_3$ | $CH_2=CH-CH_2-$ | 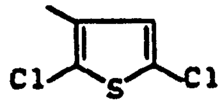 | $^1$H-NMR*) 5,35 ppm |
| 172 | $CH_3$ | $HC\equiv C-CH_2-$ | 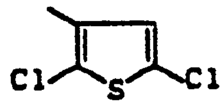 | $^1$H-NMR*) 5,4 ppm |
| 173 | $CH_3$ | $C_2H_5O-CO-CH_2-$ | 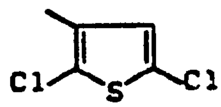 | $^1$H-NMR*) 5,4 ppm |
| 174 | $CH_3$ | O<br>‖<br>$H_2N-C-CH_2-$ | 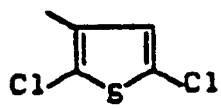 | Fp 162°C |
| 175 | $CH_3$ | O<br>‖<br>$(CH_3)_2N-C-CH_2-$ | 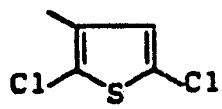 | $^1$H-NMR*) 5,3 ppm |
| 176 | $CH_3$ | O<br>‖<br>$C_2H_5NH-C-CH_2-$ | 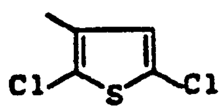 | Fp 149°C |
| 177 | $CH_3$ | O<br>‖<br>$CH_3-NH-C-CH_2-$ | 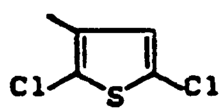 | $^1$H-NMR*) 5,4 ppm |
| 178 | $CH_3$ | $CH_3OCO-CH_2-$ | 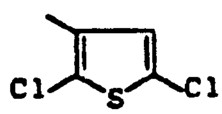 | $^1$H-NMR*) 5,3 ppm |
| 179 | $CH_3$ | 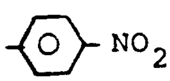 | 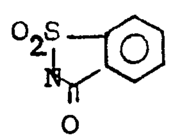 | $^1$H-NMR*) 5,45 ppm |

| Beispiel Nr. | $R^1$ | $R^2$ | Het | Physikalische Daten |
|---|---|---|---|---|
| 180 | $CH_3$ | $-CH_2OH$ | 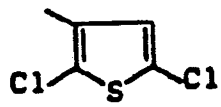 | $^1$H-NMR*) 5,3 ppm |
| 181 | H | $-CH_2-COCH_3$ | 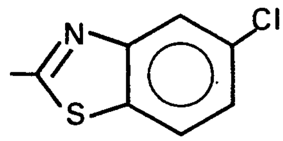 | Fp 114°C |
| 182 | H | $-CH_2-CH_2-OH$ | 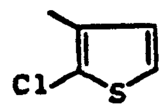 | $^1$H-NMR*) 5,5 ppm |

*) Die $^1$H-NMR-Spektren wurden aufgenommen in $CDCl_3$ mit Tetramethylsilan als innerem Standard. Angegeben sind in der Regel die chemischen Verschiebungen als δ-Werte für die Gruppierung $>C=N-O-CH_2-$.

## Herstellung der Vorprodukte der Formel (IV)

*Beispiel IV-1:*

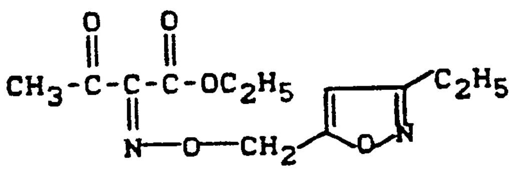

146 g (1 Mol) 2-Hydroximino-3-oxo-buttersäure-ethylester [vgl. z.B. Helv. Chem. Acta. 67, 906-915 (1984] und 159 g (1 Mol) 3-Ethyl-5-chlormethyl-isoxazol in 600 ml Acetonitril werden bei Raumtemperatur tropfenweise unter Rühren mit 140 ml (1 Mol) Triethylamin versetzt und nach beendeter Zugabe für weitere 6 Stunden bei 50°C gerührt. Zur Aufarbeitung filtriert man das ausgefallene Triethylaminhydrochlorid ab und entfernt dann das Lösungsmittel im Vakuum. Der Rückstand wird in Dichlormethan aufgenommen, mehrfach mit Wasser gewaschen, über Natriumsulfat getrocknet, im Vakuum eingeengt und im Hochvakuum destilliert.

Man erhält 157 g (59% der Theorie) an β-Keto-α-[(3-ethylisoxazol-5-yl)-methoximino]-buttersäureethylester vom Siedepunkt 80 bis 160°C/1,5 mbar.

In entsprechender Weise und gemäß den allgemeinen Angaben zur Herstellung erhält man die folgenden Alkoximino-carbonsäureester der allgemeinen Formel (IV):

```
     O  O
     ||  ||
R1-C-C-C-O-R        (IV)
        ||
        N-O-CH2-Het
```

| Beispiel Nr. | $R^1$ | $R^2$ | Het | Physikalische Daten |
|---|---|---|---|---|
| IV-2 | $CH_3$ | $C_2H_5$ | 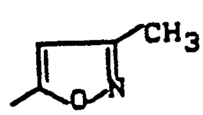 | $^1$H-NMR*) 5,33 ppm |
| IV-3 | $CH_3$ | $C_2H_5$ | 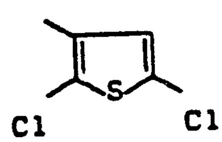 | $n_D^{20}$: 1,5321 |

*) vgl. Fußnote S. 56.

Anwendungsbeispiele:

In den folgenden Anwendungsbeispielen wurde die nachstehend aufgeführte Verbindung als Vergleichssubstanz eingesetzt:

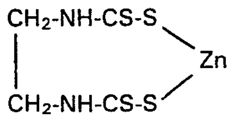

(A)

Zink-ethylen-1,2-bis-(dithiocarbamat)

*Beispiel A*

Phytophthora-Test (Tomate)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß Herstellungsbeispielen: 1, 5, 58, 68, 102, 103, 140.

*Beispiel B*

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß Herstellungsbeispielen: 3, 27, 43, 104, 110, 115, 116, 118, 119, 132.

*Beispiel C*

Pyricularia-Test (Reis)/Protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß Herstellungsbeispielen: 5, 47, 67, 101, 103, 104, 106, 116, 118, 120, 123, 126, 127, 129, 140, 150, 153.

*Beispiel D*

Pyricularia-Test (Reis)/systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß Herstellungsbeispielen: 5, 7, 8, 14, 15, 16, 150, 153.

**Patentansprüche**

1. Substituierte Pyrazolin-5-one der allgemeinen Formel (I)

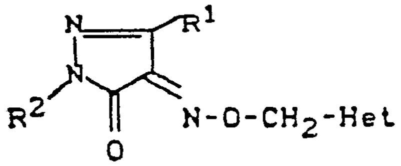

(I)

in welcher

$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Het für einen gegebenenfalls ein- bis dreifach, gleich oder verschieden substituierten und/oder benzannellierten Heterocyclus der Formel

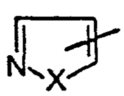 ; 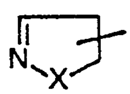 ; 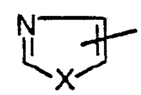 ;

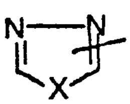 ; 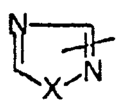 ; 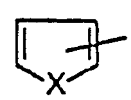 ;

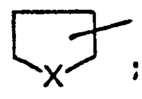 ; 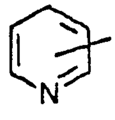 ; 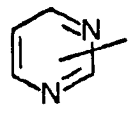 ;

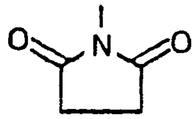 oder 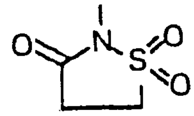

steht, wobei als Substituenten genannt seien: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht.

2. Substituierte Pyrazolin-5-one der Formel (I) gemäß Anspruch 1,

in welcher

$R^1$ und $R^2$ unabhängig voneinander jeweils für Wasserstoff, Methyl, Ethyl. n- oder i-Propyl, n-, i-, s- oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für gegebenenfalls ein- bis dreifach, gleich oder verschieden substituiertes Phenyl oder Benzyl stehen, wobei als Substituenten genannt seien: Fluor, Chlor, Brom, Iod, Cyano, Nitro, Hydroxy, Methyl, Ethyl, n- oder i-Propyl, n-, i-, s- oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

Het für einen gegebenenfalls ein- bis dreifach, gleich oder verschieden substituierten und/oder benzannellierten Heterocyclus der Formel

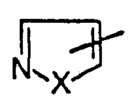 ; 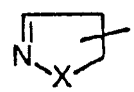 ; 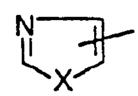 ;

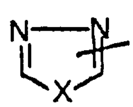 ; 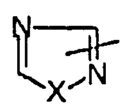 ; 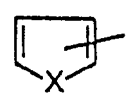 ;

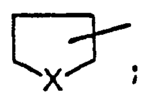 ; 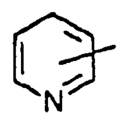 ; 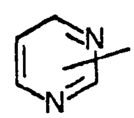 ;

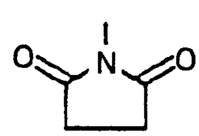 oder 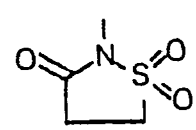

steht, wobei als Substituenten genannt seien: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht.

3. Verfahren zur Herstellung von substituierten Pyrazolin-5-onen der Formel (I)

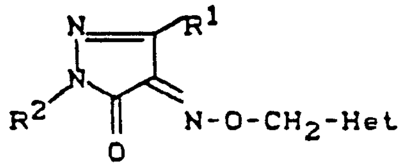

(I)

in welcher

$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Het für einen gegebenenfalls ein- bis dreifach, gleich oder verschieden substituierten und/oder benzannellierten Heterocyclus der Formel

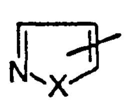 ; 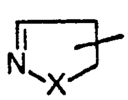 ; 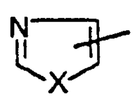 ;

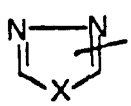 ; 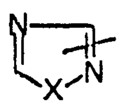 ; 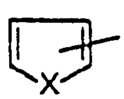 ;

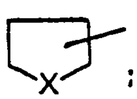 ; 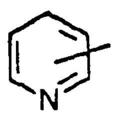 ; 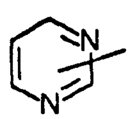 ;

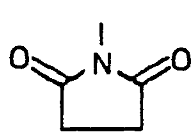 oder 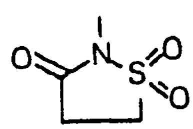

steht, wobei als Substituenten genannt seien: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht,

dadurch gekennzeichnet, daß man

(a) 4-Oximino-pyrazolin-5-one der Formel (II)

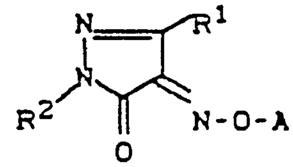

(II)

in welcher

$R^1$ und $R^2$ die oben angegebene Bedeutung haben und

A für Wasserstoff oder ein Alkalimetallkation steht,

mit Alkylierungsmitteln der Formel (III)

$$Het\text{-}CH_2\text{-}X' \qquad (III)$$

in welcher

Het die oben angegebene Bedeutung hat und

X' für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Katalysators umsetzt, oder daß man

(b) Alkoximinocarbonsäureester der Formel (IV),

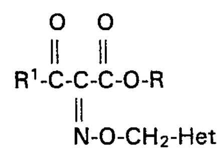

(IV)

in welcher

R für Alkyl steht und

$R^1$ und Het die oben angegebene Bedeutung haben,

mit Hydrazin-Derivaten der Formel (V)

$$R^2\text{-}NH\text{-}NH_2 \qquad (V)$$

in welcher

$R^2$ die oben angegebene Bedeutung hat

gegebenenfalls in Gegenwart eines Verdünnungsmittels umsetzt, oder daß man

(c) die nach Verfahren (a) oder nach Verfahren (b) erhältlichen 4-Alkoximino-pyrazolin-5-one der Formel (Ia),

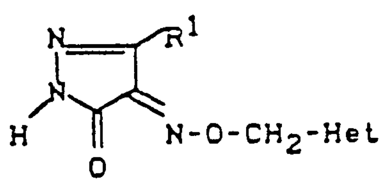

(Ia)

in welcher

$R^1$ und Het die oben angegebene Bedeutung haben, mit Alkylierungsmitteln der Formel (VI)

$$R^{2'}\text{-}Y \qquad (VI)$$

in welcher

$R^{2'}$ für jeweils geradkettiges oder verzweigtes Alkyl, Alkenyl, Alkinyl, Cyanalkyl, Hydroxyalkyl, Alkoxyalkyl, Alkylthioalkyl, Hydroxycarbonylalkyl, Alkoxycarbonylalkyl mit jeweils bis zu 8 Kohlenstoffatomen in den einzelnen Alkyl- bzw. Alkenyl- oder Alkinylteilen, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen in Arylteil, wobei als Substituenten in dem Arylteil jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Y für eine elektronenanziehende Abgangsgruppe steht,

gegebenenfalls in Gegenwart eines Verdünnungsmittels, gegebenenfalls in Gegenwart eines Säurebindemittels und gegebenenfalls in Gegenwart eines Katalysators umsetzt.

4. Schädlingsbekämpfungsmittel, gekennzeichnet durch einen Gehalt an mindestens einem substituierten Pyrazolin-5-on der Formel (I) gemäß den Ansprüchen 1 bis 3.

5. Verwendung von substituierten Pyrazolin-5-onen der Formel (I) gemäß den Ansprüchen 1 bis 3 zur Bekämpfung von Schädlingen.

6. Verfahren zur Bekämpfung von Schädlingen, dadurch gekennzeichnet, daß man substituierte Pyrazolin-5-one der Formel (I) gemäß den Ansprüchen 1 bis 3 auf Schädlinge und/oder ihren Lebensraum einwirken läßt.

7. Verfahren zur Herstellung von Schädlingsbekämpfungsmittels, dadurch gekennzeichnet, daß man substituierte Pyrazolin-5-one der Formel (I) gemäß den Ansprüchen 1 bis 3 mit Streckmitteln und/oder oberflächenaktiven Mitteln vermischt.

8. Alkoximinocarbonsäureester der Formel (IV),

$$R^1\text{-}\overset{\overset{O}{\|}}{C}\text{-}\underset{\underset{N\text{-}O\text{-}CH_2\text{-}Het}{\|}}{C}\text{-}\overset{\overset{O}{\|}}{C}\text{-}O\text{-}R \qquad (IV)$$

in welcher

R für Alkyl steht,

$R^1$ für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Het für einen gegebenenfalls ein- bis dreifach, gleich oder verschieden substituierten und/oder benzannellierten Heterocyclus der Formel

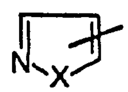 ; 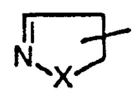 ; 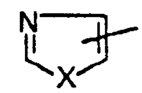 ;

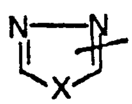 ; 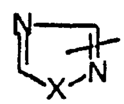 ; 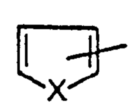 ;

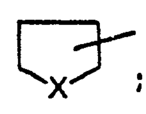 ; 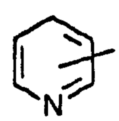 ; 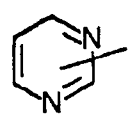 ;

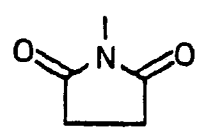 oder 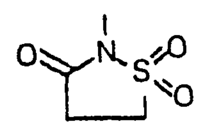

steht, wobei als Substituenten genannt seien: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht.

9. Verfahren zur Herstellung von Alkoximinocarbonsäureestern der Formel (IV)

$$R^1\text{-}\overset{\overset{O}{\|}}{C}\text{-}\underset{\underset{N\text{-}O\text{-}CH_2\text{-}Het}{\|}}{C}\text{-}\overset{\overset{O}{\|}}{C}\text{-}O\text{-}R \qquad (IV)$$

in welcher

R für Alkyl steht,

$R^1$ für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für geradkettiges oder verzweigtes, gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder für gegebenenfalls einfach oder mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, geradkettiges oder verzweigtes Halogenalkyl, Halogenalkoxy oder Halogenalkylthio mit jeweils bis zu 4 Kohlenstoffatomen und bis zu 9 gleichen oder verschiedenen Halogenatomen oder Phenyl und

Het für einen gegebenenfalls ein- bis dreifach, gleich oder verschieden substituierten und/oder benzannellierten Heterocyclus der Formel

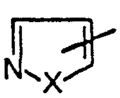 ; 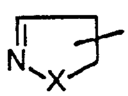 ; 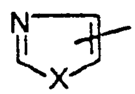 ;

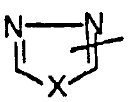 ; 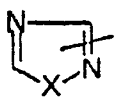 ; 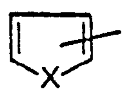 ;

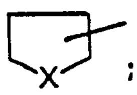 ; 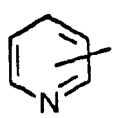 ; 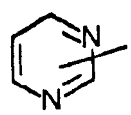 ;

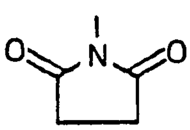 oder 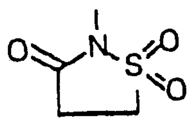

steht, wobei als Substituenten genannt seien: Fluor, Chlor, Brom, Methyl, Ethyl, n- oder i-Propyl, n-, i-, s- oder t-Butyl, Methoxy, Ethoxy, Trifluormethyl oder gegebenenfalls ein- bis dreifach, gleich oder verschieden durch Chlor, Nitro, Methyl und/oder Methoxy substituiertes Phenyl und wobei

X jeweils für Sauerstoff oder Schwefel steht,

dadurch gekennzeichnet, daß man Hydroximinocarbonsäureester der Formel (XI),

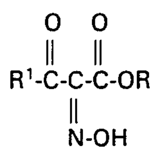 (XI)

in welcher

R und $R^1$ die oben angegebene Bedeutung hat,

mit Alkylierungsmitteln der Formel (III)

$$\text{Het-CH}_2\text{-X}' \qquad \text{(III)}$$

in welcher

Het die oben angegebene Bedeutung hat und

X' für eine elektronenanziehende Abgangsgruppe steht,

gegebenenfalls in Gegenwart eines Verdünnungsmittels sowie gegebenenfalls in Gegenwart eines Säurebindemittels bei Temperaturen zwischen +10°C und +80°C umsetzt.

## Claims

1. Substituted pyrazolin-5-ones of the general formula (I)

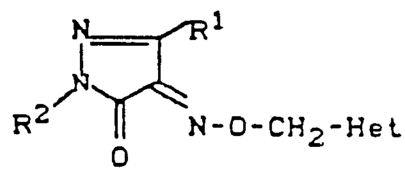 (I)

in which

$R^1$ and $R^2$ independently of one another each represent hydrogen, or represent in each case straight-chain or branched alkyl, alkenyl, alkinyl, cyanoalkyl, hydroxyalkyl, alkoxyalkyl, alkylthioalkyl, alkoxycarbonyl, hydroxycarbonylalkyl, alkoxy-carbonylalkyl, aminocarbonylalkyl, alkylaminocarbonylalkyl or dialkylaminocarbonylalkyl with in each case up to 8 carbon atoms in the individual alkyl, alkenyl or alkinyl parts, or represent oxiranylalkyl with 1 to 4 carbon atoms in the straight-chain or branched alkyl part, or represent 1,1-dioxotetrahydrothienyl, or represent straight-chain or branched aralkyl which has 1 to 4 carbon atoms in the alkyl part and 6 to 10 carbon atoms in the aryl part and is optionally monosubstituted or polysubstituted by identical or different substituents, or represent aryl which has 6 to 10 carbon atoms and is optionally monosubstituted or polysubstituted by identical or different substituents, possible substituents in the aryl parts in each case being: halogen, cyano, nitro, hydroxyl, in each case straight-chain or branched alkyl, alkoxy, dioxyalkylene, alkylcarbonyloxy and alkylthio with in each case up to 4 carbon atoms, straight-chain or branched halogenoalkyl, halogenoalkoxy or halogenoalkylthio with in each case up to 4 carbon atoms and up to 9 identical or different halogen atoms or phenyl and

Het represents a heterocycle of the formula

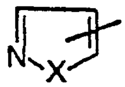 ; 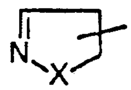 ; 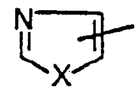 ;
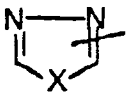 ; 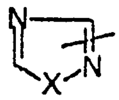 ; 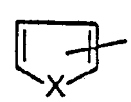 ;
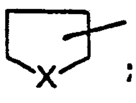 ; 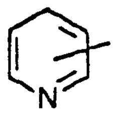 ; 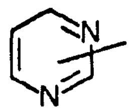 ;
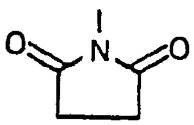 or 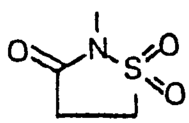

which is optionally monosubstituted to trisubstituted by identical or different substituents and/or benzo-fused, possible substituents which may be mentioned being: fluorine, chlorine, bromine, methyl, ethyl, n- or i-propyl, n-, i-, s- or t-butyl, methoxy, ethoxy, trifluoromethyl, or phenyl which is optionally monosubstituted to trisubstituted by identical or different substituents from the group comprising chlorine, nitro, methyl and/or methoxy and wherein

X in each case represents oxygen or sulfur.

2. Substituted pyrazolin-5-ones of the formula (I) according to Claim 1,

in which

$R^1$ and $R^2$ independently of one another in each case represent hydrogen, methyl, ethyl, n- or i-propyl, n- i-, s- or t-butyl, allyl, butenyl, propargyl, cyanomethyl, cyanoethyl, hydroxymethyl, hydroxyethyl, methoxymethyl, methoxyethyl, ethoxymethyl, ethoxyethyl, methylthiomethyl, methoxycarbonyl, ethoxycarbonyl, hydroxycarbonylmethyl, hydroxycarbonylethyl, methoxycarbonylmethyl, methoxycarbonylethyl, ethoxycarbonylmethyl, ethoxycarbonylethyl, aminocarbonylmethyl, methylaminocarbonylmethyl, ethylaminocarbonylmethyl, dimethylaminocarbonylmethyl, diethylaminocarbonylmethyl, aminocarbonylethyl, oxiranylmethyl or oxiranylethyl, or represent 1,1-dioxotetrahydrothien-3-yl, or represent phenyl or benzyl which is optionally monosubstituted to trisubstituted by identical or different substituents, possible substituents which may be mentioned being: fluorine, chlorine, bromine, iodine, cyano, nitro, hydroxyl, methyl, ethyl, n- or i-propyl, n-, i-, s- or t-butyl, methoxy, ethoxy, dioxymethylene, dioxyethylene, methylthio, ethylthio, acetoxy or propionyloxy, chloromethyl, dichloromethyl, trichloromethyl, trifluoromethyl, dichlorofluoromethyl, chlorodifluoromethyl, trifluoromethoxy, trifluoromethylthio and phenyl and

Het represents a heterocycle of the formula

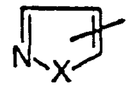 ; 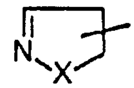 ; 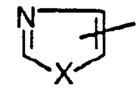 ;
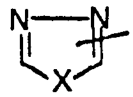 ; 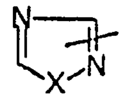 ; 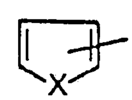 ;
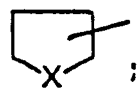 ; 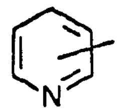 ; 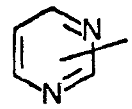 ;
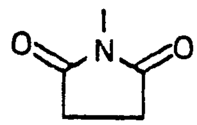 or 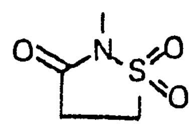

which is optionally monosubstituted to trisubstituted by identical or different substituents and/or benzo-fused, substituents which may be mentioned being: fluorine, chlorine, bromine, methyl, ethyl, n- or i-propyl, n-, i-, s- or t-butyl, methoxy, ethoxy, trifluoromethyl or phenyl which is optionally monosubstituted to trisubstituted by identical or different substituents from the group comprising chlorine, nitro, methyl and/or methoxy and wherein

X in each case represents oxygen or sulfur.

3. Process for the preparation of substituted pyrazolin-5-ones of the formula (I)

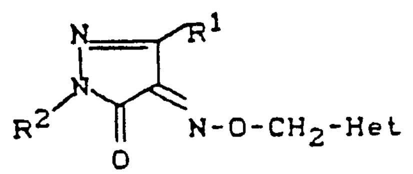

(I)

in which

$R^1$ and $R^2$ independently of one another each represent hydrogen, or represent in each case straight-chain or branched alkyl, alkenyl, alkinyl, cyanoalkyl, hydroxyalkyl, alkoxyalkyl, alkylthioalkyl, alkoxycarbonyl, hydroxycarbonylalkyl, alkoxycarbonylalkyl, aminocarbonylalkyl, alkylaminocarbonylalkyl or dialkylaminocarbonylalkyl with in each case up to 8 carbon atoms in the individual alkyl, alkenyl or alkinyl parts, or represent oxiranylalkyl with 1 to 4 carbon atoms in the straight-chain or branched alkyl part, or represent 1,1-dioxotetrahydrothienyl, or represent straight-chain or branched aralkyl which has 1 to 4 carbon atoms in the alkyl part and 6 to 10 carbon atoms in the aryl part and is optionally monosubstituted or polysubstituted by identical or different substituents, or represent aryl which has 6 to 10 carbon atoms and is optionally monosubstituted or polysubstituted by identical or different substituents, possible substituents in the aryl parts in each case being: halogen, cyano, nitro, hydroxyl, in each case straight-chain or branched

alkyl, alkoxy, dioxyalkylene, alkylcarbonyloxy and alkylthio with in each case up to 4 carbon atoms, straight-chain or branched halogenoalkyl, halogenoalkoxy or halogenoalkylthio with in each case up to 4 carbon atoms and up to 9 identical or different halogen atoms or phenyl and

Het represents a heterocycle of the formula

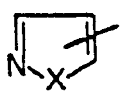 ; 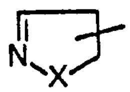 ; 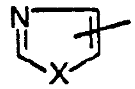 ;

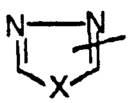 ; 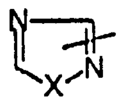 ; 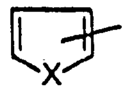 ;

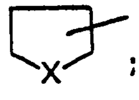 ; 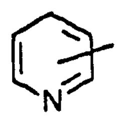 ; 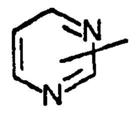 ;

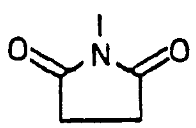 or 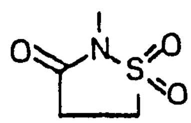

which is optionally monosubstituted to trisubstituted by identical or different substituents and/or benzo-fused, possible substituents which may be mentioned being: fluorine, chlorine, bromine, methyl, ethyl, n- or i-propyl, n-, i-, s- or t-butyl, methoxy, ethoxy, trifluoromethyl, or phenyl which is optionally monosubstituted to trisubstituted by identical or different substituents from the group comprising chlorine, nitro, methyl and/or methoxy and wherein

X in each case represents oxygen or sulfur,

characterized in that

(a) 4-oximino-pyrazolin-5-ones of the formula (II)

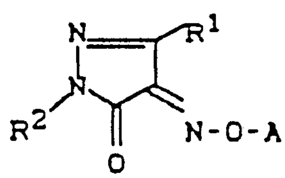 (II)

in which

$R^1$ and $R^2$ have the abovementioned meaning and

A represents hydrogen or an alkali metal cation, are reacted with alkylating agents of the formula (III)

$$Het\text{-}CH_2\text{-}X' \quad (III)$$

in which

Het has the abovementioned meaning and

X' represents an electron-withdrawing leaving group,

if appropriate in the presence of a diluent, if appropriate in the presence of an acid-binding agent and if appropriate in the presence of a catalyst, or in that

(b) the alkoximinocarboxylic acid esters of the formula (IV)

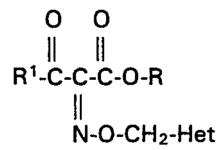 (IV)

in which

R represents alkyl and

$R^1$ and Het have the abovementioned meaning,

are reacted with hydrazine derivatives of the formula (V)

$$R^2\text{-NH-NH}_2 \quad (V)$$

in which

$R^2$ has the abovementioned meaning, if appropriate in the presence of a diluent, or in that

(c) the 4-alkoximino-pyrazolin-5-ones obtainable by process (a) or by process (b), of the formula (Ia)

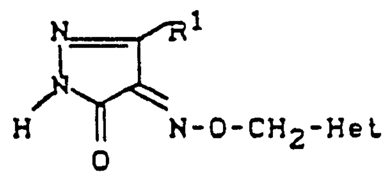 (Ia)

in which

$R^1$ and Het have the abovementioned meaning, are reacted with alkylating agents of the formula (VI)

$$R^{2'}\text{-Y} \quad (VI)$$

in which

$R^{2'}$ represents in each case straight-chain or branched alkyl, alkenyl, alkinyl, cyanoalkyl, hydroxyalkyl, alkoxyalkyl, alkylthioalkyl, hydroxycarbonylalkyl, alkoxycarbonylalkyl with in each case up to 8 carbon atoms in the individual alkyl, alkenyl or alkinyl parts, or represents 1,1-dioxotetrahydrothienyl, or represents straight-chain or branched aralkyl which has 1 to 4 carbon atoms in the alkyl part and 6 to 10 carbon atoms in the aryl part and is optionally monosubstituted or polysubstituted by identical or different substituents, possible substituents in the aryl parts in each case being: halogen, cyano, nitro, hydroxyl, in each case straight-chain or branched alkyl, alkoxy, dioxyalkylene, alkylcarbonyloxy and alkylthio with in each case up to 4 carbon atoms, straight-chain or branched halogenalkyl, halogenalkoxy or halogenoalkylthio with in each case up to 4 carbon atoms and up to 9 identical or different halogen atoms or phenyl and

Y represents an electron-withdrawing leaving group,

if appropriate in the presence of a diluent, if appropriate in the presence of an acid-binding agent and if appropriate in the presence of a catalyst.

4. Agents for combating pests, characterized in that they contain at least one substituted pyrazolin-5-one of the formula (I) according to Claim 1 to 3.

5. Use of substituted pyrazolin-5-ones of the formula (I) according to Claims 1 to 3 for combating pests.

6. Method of combating pests, characterized in that substituted pyrazolin-5-ones of the formula (I) according to Claims 1 to 3 are allowed to act on pests and/or their environment.

7. Process for the preparation of agents for combating pests, characterized in that substituted pyrazolin-5-ones of the formula (I) according to Claims 1 to 3 are mixed with extenders and/or surface-active agents.

8. Alkoximinocarboxylic acid esters of the formula (IV)

O O
|| ||
$R^1$-C-C-C-O-R (IV)
||
N-O-$CH_2$-Het

in which

R represents alkyl,

$R^1$ represents hydrogen, or represents in each case straight-chain or branched alkyl, alkenyl, alkinyl, cyanoalkyl, hydroxyalkyl, alkoxyalkyl, alkylthioalkyl, alkoxycarbonyl, hydroxycarbonylalkyl, alkoxycarbonylalkyl, aminocarbonylalkyl, alkylaminocarbonylalkyl or dialkylaminocarbonylalkyl with in each case up to 8 carbon atoms in the individual alkyl, alkenyl or alkinyl parts, or represents oxiranylalkyl with 1 to 4 carbon atoms in the straight-chain or branched alkyl part, or represents 1,1-dioxotetrahydrothienyl, or represents straight-chain or branched aralkyl which has 1 to 4 carbon atoms in the alkyl part and 6 to 10 carbon atoms in the aryl part and is optionally monosubstituted or polysubstituted by identical or different substituents, or represent aryl which has 6 to 10 carbon atoms and is optionally monosubstituted or polysubstituted by identical or different substituents, possible substituents in the aryl parts in each case being: halogen, cyano, nitro, hydroxyl, in each case straight-chain or branched alkyl, alkoxy, dioxyalkylene, alkylcarbonyloxy and alkylthio with in each case up to 4 carbon atoms, straight-chain or branched halogenoalkyl, halogenoalkoxy or halogenoalkylthio with in each case up to 4 carbon atoms and up to 9 identical or different halogen atoms or phenyl and

Het represents a heterocycle of the formula

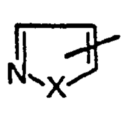 ; 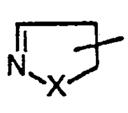 ; 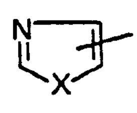 ;

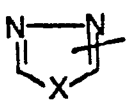 ; 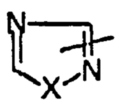 ; 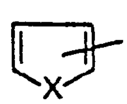 ;

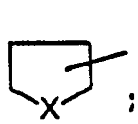 ; 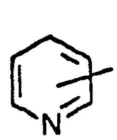 ; 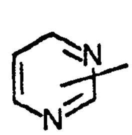 ;

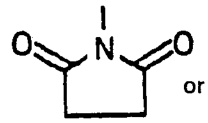 or 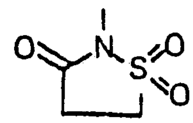

which is optionally monosubstituted to trisubstituted by identical or different substituents and/or benzofused, possible substituents which may be mentioned being: fluorine, chlorine, bromine, methyl, ethyl, n- or i-propyl, n-, i-, s- or t-butyl, methoxy, ethoxy, trifluoromethyl, or phenyl which is optionally monosubstituted to trisubstituted by identical or different substituents from the group comprising chlorine, nitro, methyl and/or methoxy and wherein

X in each case represents oxygen or sulfur.

9. Process for the preparation of alkoximinocarboxylic acid esters of the formula (IV)

O O
|| ||
$R^1$-C-C-C-O-R (IV)
||
N-O-$CH_2$-Het

in which

R represents alkyl,

$R^1$ represents hydrogen, or represents in each case straight-chain or branched alkyl, alkenyl, alkinyl, cyanoalkyl, hydroxyalkyl, alkoxyalkyl, alkylthioalkyl, alkoxycarbonyl, hydroxycarbonylalkyl, alkoxycarbonylalkyl, aminocarbonylalkyl, alkylaminocarbonylalkyl or dialkylaminocarbonylalkyl with in each case up to 8 carbon atoms in the individual alkyl, alkenyl or alkinyl parts, or represent oxiranylalkyl with 1 to 4 carbon atoms in the straight-chain or branched alkyl part, or represents 1,1-dioxotetrahydrothienyl, or represents straight-chain or branched aralkyl which has 1 to 4 carbon atoms in the alkyl part and 6 to 10 carbon atoms in the aryl part and is optionally monosubstituted or polysubstituted by identical or different substituents, or represent aryl which has 6 to 10 carbon atoms and is optionally monosubstituted or polysubstituted by identical or different substituents, possible substituents in the aryl parts in each case being: halogen, cyano, nitro, hydroxyl, in each case straight-chain or branched alkyl, alkoxy, dioxyalkylene, alkylcarbonyloxy and alkylthio with in each case up to 4 carbon atoms, straight-chain or branched halogenoalkyl, halogenoalkoxy or halogenoalkylthio with in each case up to 4 carbon atoms and up to 9 identical or different halogen atoms or phenyl and

Het represents a heterocycle of the formula

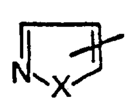 ; 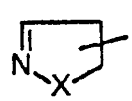 ; 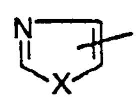 ;

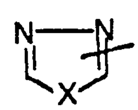 ; 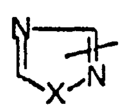 ; 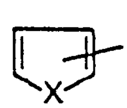 ;

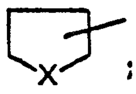 ; 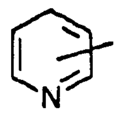 ; 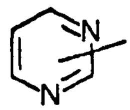 ;

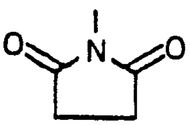 or 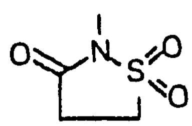

which is optionally monosubstituted to trisubstituted by identical or different substituents and/or benzo-fused, possible substituents which may be mentioned being: fluorine, chlorine, bromine, methyl, ethyl, n- or i-propyl, n-, i-, s- or t-butyl, methoxy, ethoxy, trifluoromethyl, or phenyl which is optionally monosubstituted to trisubstituted by identical or different substituents from the group comprising chlorine, nitro, methyl and/or methoxy and wherein

X in each case represents oxygen or sulfur,

characterized in that hydroximinocarboxylic acid esters of the formula (XI)

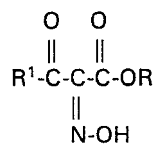 (XI)

in which

R and $R^1$ have the abovementioned meaning, are reacted with alkylating agents of the formula (III)

$$Het\text{-}CH_2\text{-}X' \qquad (III)$$

in which

Het has the abovementioned meaning and

X' represents an electron-withdrawing leaving group,

if appropriate in the presence of a diluent and if appropriate in the presence of an acid-binding agent, at temperature between +10°C and +80°C.

**Revendications**

1. Pyrazoline-5-ones substituées de formule générale I

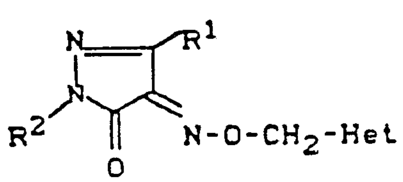 (I)

dans laquelle

$R^1$ et $R^2$ représentent chacun, indépendamment l'un de l'autre, l'hydrogène, un groupe alkyle, alcényle, alcynyle, cyanalkyle, hydroxyalkyle, alcoxyalkyle, alkylthioalkyle, alcoxycarbonyle, hydroxycarbonylalkyle, alcoxycarbonylalkyle, aminocarbonylalkyle, alkylaminocarbonylalkyle ou dialkylaminocarbonylalkyle, chacun à chaîne droite ou ramifiée et contenant chacun jusqu'à 8 atomes de carbone dans chacune des parties alkyle, alcényle ou alcynyle, un groupe oxirannylakyle contenant 1 à 4 atomes de carbone dans la partie alkyle à chaîne droite ou ramifiée, un groupe 1,1-dioxotétrahydrothienyle, un groupe aralkyle à chaîne droite ou ramifiée, portant éventuellement un ou plusieurs substituants identiques ou différents et contenant 1 à 4 atomes de carbone dans la partie alkyle et 6 à 10 atomes de carbone dans la partie aryle, ou un groupe aryle portant éventuellement un ou plusieurs substituants identiques ou différents et contenant 6 à 10 atomes de carbone, les substituants des parties aryle étant: des halogènes, des groupes cyano, nitro, hydroxy, des groupes alkyle, alcoxy, dioxyalkylène, alkylcarbonyloxy et alkylthio chacun à chaîne droite ou ramifiée et contenant chacun jusqu'à 4 atomes de carbone, des groupes halogénoalkyle, halogénoalcoxy ou halogénoalkylthio à chaîne droite ou ramifiée contenant chacun jusqu'à 4 atomes de carbone et jusqu'à 9 atomes d'halogènes identiques ou différents ou des groupes phényle, et

Het représente un hétérocycle portant éventuellement 1 à 3 substituants identiques ou différents et/ou condensé sur un noyau benzénique, de formule

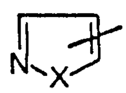 ; 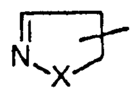 ; 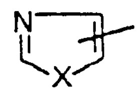 ;

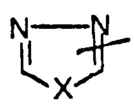 ; 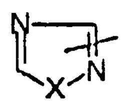 ; 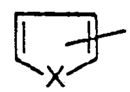 ;

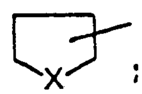 ; 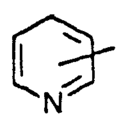 ; 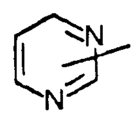 ;

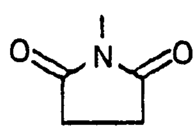 ou 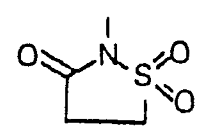

les substituants en question étant: le fluor, le chlore, le brome, des groupes méthyle, éthyle, n- ou isopropyle, n-, iso-, sec- ou tert-butyle, méthoxy, éthoxy, trifluorométhyle ou phényle portant éventuellement 1 à 3 substituants identiques ou différents choisis parmi le chlore, les groupes nitro, méthyle et/ou méthoxy, et

X dans chaque cas, représente l'oxygène ou le soufre.

2. Pyrazoline-5-ones substituées de formule 1 selon la revendication 1, dans lesquelles

$R^1$ et $R^2$ représentent chacun, indépendamment l'un de l'autre, l'hydrogène, un groupe méthyle, éthyle, n- ou iso-propyle, n-, iso-, sec- ou tert-butyle, allyle, butényle, propargyle, cyanométhyle, cyanéthyle, hydroxyméthyle, hydroxyéthyle, méthoxy-

méthyle, méthoxyéthyle, éthoxyméthyle, éthoxyéthyle, méthylthiométhyle, méthoxycarbonyle, éthoxycarbonyle, hydroxycarbonylméthyle, hydroxycarbonyléthyle, méthoxycarbonylméthyle, méthoxycarbonyléthyle, éthoxycarbonylméthyle, éthoxycarbonyléthyle, aminocarbonylméthyle, méthylaminocarbonylméthyle, éthaylaminocarbonylméthyle, diméthylaminocarbonylméthyle, diéthylaminocarbonylméthyle, aminocarbonyléthyle, oxiranylméthyle, oxirannyléthyle, un groupe 1,1-dioxotétrahydrothiène-3-yle ou un groupe phényle ou benzyle portant éventuellement un à trois substituants identiques ou différents, ces substituants étant: le fluor, le chlore, le brome, l'iode, des groupes cyano, nitro, hydroxy, méthyle, éthyle, n- ou isoproyle, n-, iso-, sec- ou tert-butyle, méthoxy, éthoxy, dioxyméthylène, dioxyéthylène, méthylthio, éthylthio, acétoxy ou propionyloxy, chlorométhyle, dichlorométhyle, trichlorométhyle, trifluorométhyle, dichlorofluorométhyle, chlorodifluorométhyle, trifluorométhoxy, trifluorométhylthio ou phényle, et

Het représente un hétérocycle portant éventuellement 1 à 3 substituants identiques ou différents et/ou condensé sur un noyau benzénique, de formule

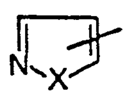 ; 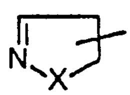 ; 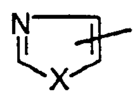 ;
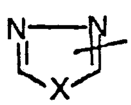 ; 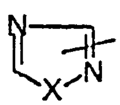 ; 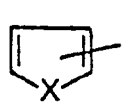 ;
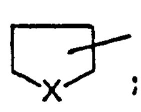 ; 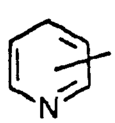 ; 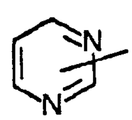 ;

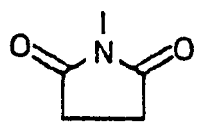 ou 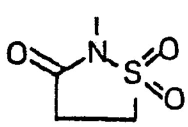

les substituants en question étant: le fluor, le chlore, le brome, des groupes méthyle, éthyle, n- ou isopropyle, n-, iso-, sec- ou tert-butyle, méthoxy, éthoxy, trifluorométhyle ou phényle portant éventuellement 1 à 3 substituants identiques ou différents choisis parmi le chlore, les groupes nitro, méthyle et/ou méthoxy, et

X représente dans chaque cas l'oxygène ou le soufre.

3. Procédé de préparation des pyrazoline-5-ones substituées de formule I

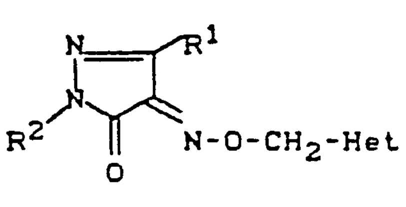 (I)

dans laquelle

$R^1$ et $R^2$ représentent chacun, indépendamment l'un de l'autre, l'hydrogène, un groupe alkyle, alcényle, alcynyle, cyanalkyle, hydroxyalkyle, alcoxyalkyle, alkylthioalkyle, alcoxycarbonyle, hydroxycarbonylalkyle, alcoxycarbonylalkyle, aminocarbonylalkyle, alkylaminocarbonylalkyle ou dialkylaminocarbonylalkyle, chacun à chaîne droite ou ramifiée et contenant jusqu'à 8 atomes de carbone dans chacune des parties alkyle, alcényle ou alcynyle, un groupe oxirannylakyle contenant 1 à 4 atomes de carbone dans la partie alkyle à chaîne droite ou ramifiée, un groupe 1,1-dioxotétrahydrothiényle, un groupe aralkyle à chaîne droite ou ramifiée, portant éventuellement un ou plusieurs substituants identiques ou différents et contenant 1 à 4 atomes de carbone dans la partie alkyle et 6 à 10 atomes de carbone dans la partie aryle, ou un groupe aryle portant éventuellement un ou plusieurs substituants identiques ou différents et contenant 6 à 10 atomes de carbone, les substituants des parties aryle étant: des halogènes, des groupes cyano, nitro, hydroxy, des groupes alkyle, alcoxy, dioxyalkylène, alkylcarbonyloxy et alkylthio chacun à chaîne droite ou ramifiée et contenant chacun jusqu'à 4 atomes de carbone, des groupes halogénoalkyle, halogénoalcoxy ou halogénoalkylthio à chaîne droite ou ramifiée contenant chacun jusqu'à 4 atomes de carbone et jusqu'à 9 atomes d'halogènes identiques ou différents ou des groupes phényle, et

Het représente un hétérocycle portant éventuellement 1 à 3 substituants identiques ou différents et/ou condensé sur un noyau benzénique, de formule

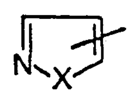 ; 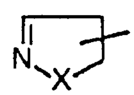 ; 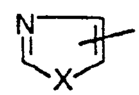 ;
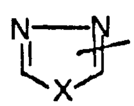 ; 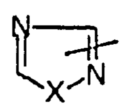 ; 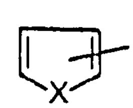 ;
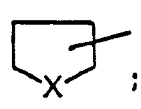 ; 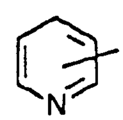 ; 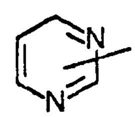 ;

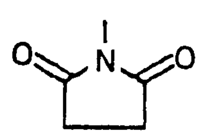 ou 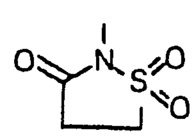

les substituants en question étant: le fluor, le chlore, le brome, des groupes méthyle, éthyle, n- ou isopropyle, n-, iso-, sec- ou tert-butyle, méthoxy, éthoxy, trifluorométhyle ou phényle portant éventuellement 1 à 3 substituants identiques ou différents choisis parmi le chlore, les groupes nitro, méthyle et/ou méthoxy, et

X dans chaque cas, représente l'oxygène ou le soufre,

caractérisé en ce que:

(a) on fait réagir des 4-oximino-pyrazoline-5-ones de formule II

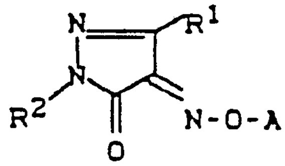

(II)

dans laquelle

$R^1$ et $R^2$ ont les significations indiquées ci-dessus, et

A représente l'hydrogène ou un cation de métal alcalin, avec des agents alkylants de formule III

$$Het\text{-}CH_2\text{-}X' \quad (III)$$

dans laquelle

Het a les significations indiquées ci-dessus, et

X' représente un groupe éliminable électrophile,

éventuellement en présence d'un diluant, éventuellement en présence d'un accepteur d'acide et éventuellement en présence d'un catalyseur, ou bien

(b) on fait réagir des esters d'acides alcoximinocarboxyliques de formule IV

O O
|| ||
$R^1$-C-C-C-O-R (IV)
||
N-O-$CH_2$-Het

dans laquelle

R représente un groupe alkyle, et

$R^1$ et Het ont les significations indiquées ci-dessus, avec des dérivés de l'hydrazine de formule V

$$R^2\text{-}NH\text{-}NH_2 \quad (V)$$

dans laquelle

$R^2$ a les significations indiquées ci-dessus,

éventuellement en présence d'un accepteur d'acide, ou bien

(c) on fait réagir les 4-alcoximino-pyrazoline-5-ones de formule Ia

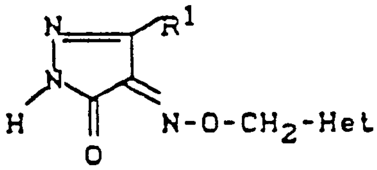

(Ia)

dans laquelle

$R^1$ et Het ont les significations indiquées ci-dessus, obtenues par le procédé (a) ou par le procédé (b), avec des agents alkylants de formule VI

$$R^{2'}\text{-}Y \quad (VI)$$

dans laquelle

$R^{2'}$ représente un groupe alkyle, alcényle, alcynyle, cyanalkyle, hydroxyalkyle, alcoxyalkyle, alkylthioalkyle, hydroxycarbonylalkyle, alcoxycarbonylalkyle, chacun à chaîne droite ou ramifiée et contenant chacun jusqu'à 8 atomes de carbone dans les diverses parties alkyle, alcényle ou alcynyle, un groupe 1,1-dioxotétrahydrothiényle, un groupe aralkyle à chaîne droite ou ramifiée portant éventuellement un ou plusieurs substituants identiques ou différents et contenant 1 à 4 atomes de carbone dans la partie alkyle et 6 à 10 atomes de carbone dans la partie aryle, les substituants de la partie aryle étant les suivants: les halogènes, les groupes cyano, nitro, hydroxy, les groupes alkyle, alcoxy, dioxyalkylène. alkylcarbonyloxy et alkylthio chacun à chaîne droite ou ramifiée et contenant chacun jusqu'à 4 atomes de carbone, les groupes halogénoalkyle, halogénoalcoxy ou halogénoalkylthio à chaîne droite ou ramifiée contenant chacun jusqu'à 4 atomes de carbone et jusqu'à 9 atomes d'halogènes identiques ou différents, ou les groupes phényle, et

Y représente un groupe éliminable électrophile,

éventuellement en présence d'un diluant, éventuellement en présence d'un capteur d'acide et éventuellement en présence d'un catalyseur.

4. Produit pesticide, caractérisé en ce qu'il contient au moins une pyrazoline-5-one substituée de formule I, selon les revendications 1 à 3.

5. Utilisation des pyrazoline-5-ones substituées de formule I, selon les revendications 1 à 3 dans la lutte contre les parasites.

6. Procédé pour combattre les parasites, caractérisé en ce que l'on fait agir des pyrazoline-5-ones substituées de formule I, selon les revendications 1 à 3 sur les parasites et/ou leur habitat.

7. Procédé de préparation de produits pesticides, caractérisé en ce que l'on mélange des pyrazoline-5-ones substituées de formule I, selon les revendications 1 à 3 avec des diluants et/ou des agents tensioactifs.

8. Esters d'acides alcoximinocarboxyliques de formule IV

O O
|| ||
$R^1$-C-C-C-O-R (IV)
||
N-O-$CH_2$-Het

danns laquelle

R représente un groupe alkyle,

$R^1$ représente l'hydrogène, un groupe alkyle, alcényle, alcynyle, cyanalkyle, hydroxyalkyle, alcoxyalkyle, alkylthioalkyle, alcoxycarbonyle, hydroxycarbonylalkyle, alcoxycarbonylalkyle, aminocarbonylalkyle, alkylaminocarbonylalkyle ou dialkylaminocarbonylalkyle, chacun à chaîne droite ou ramifiée et contenant chacun jusqu'à 8 atomes de carbone dans les diverses parties alkyle, alcényle ou alcynyle, un groupe oxirannylakyle contenant 1 à 4 atomes de carbone dans la partie alkyle à chaîne droite ou ramifiée, un groupe 1,1-dioxotétrahydrothienyle, un groupe aralkyle à chaîne droite ou ramifiée, portant éventuellement un ou plusieurs substituants identiques ou différents et contenant 1 à 4 atomes de carbone dans la partie alkyle et 6 à 10 atomes de carbone dans la partie aryle, ou un groupe aryle portant éventuellement un ou plusieurs substituants identiques ou différents et contenant 6 à 10 atomes de carbone, les substituants des parties aryle étant: les halogènes, des groupes cyano, nitro, hydroxy, des groupes alkyle, alcoxy, dioxyalkylène, alkylcarbonyloxy et alkylthio chacun à chaîne droite ou ramifiée et contenant chacun jusqu'à 4 atomes de carbone, des groupes halogénoalkyle, halogénoalcoxy ou halogénoalkylthio à chaîne droite ou ramifiée contenant chacun jusqu'à 4 atomes de carbone et jusqu'à 9 atomes d'halogènes identiques ou différents, ou des groupes phényle, et

Het représente un hétérocycle portant éventuellement 1 à 3 substituants identiques ou différents et/ou condensé sur un noyau benzénique, de formule

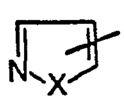 ; 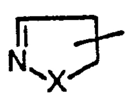 ; 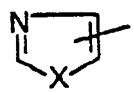 ;

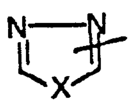 ; 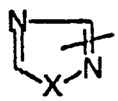 ; 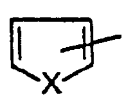 ;

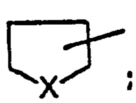 ; 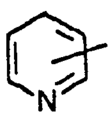 ; 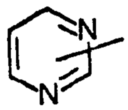 ;

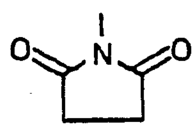 ou 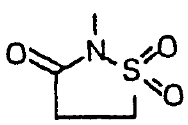

les substituants en question étant: le fluor, le chlore, le brome, des groupes méthyle, éthyle, n- ou isopropyle, n-, iso-, sec- ou tert-butyle, méthoxy, éthoxy, trifluorométhyle ou des groupes phényle portant éventuellement 1 à 3 substituants identiques ou différents choisis parmi le chlore, les groupes nitro, méthyle et/ou méthoxy, et

X représente dans chaque cas l'oxygène ou le soufre.

9. Procédé de préparation des esters d'acides alcoximinocarboxyliques de formule IV

```
     O  O
     ||  ||
R¹-C-C-C-O-R        (IV)
        ||
        N-O-CH2-Het
```

dans laquelle

R représente un groupe alkyle,

$R^1$ représente l'hydrogène, un groupe alkyle, alcényle, alcynyle, cyanalkyle, hydroxyalkyle, alcoxyalkyle, alkylthioalkyle, alcoxycarbonyle, hydroxycarbonylalkyle, alcoxycarbonylalkyle, aminocarbonylalkyle, alkylaminocarbonylalkyle ou dialkylaminocarbonylalkyle, chacun à chaîne droite ou ramifiée et contenant chacun jusqu'à 8 atomes de carbone dans les diverses parties alkyle, alcényle ou alcynyle, un groupe oxirannylakyle contenant 1 à 4 atomes de carbone dans la partie alkyle à chaîne droite ou ramifiée, un groupe 1,1-dioxotétrahydrothienyle, un groupe aralkyle à chaîne droite ou ramifiée, portant éventuellement un ou plusieurs substituants identiques ou différents et contenant 1 à 4 atomes de carbone dans la partie alkyle et 6 à 10 atomes de carbone dans la partie aryle, ou un groupe aryle portant éventuellement un ou plusieurs substituants identiques ou différents et contenant 6 à 10 atomes de carbone, les substituants des parties aryle étant: les halogènes, les groupes cyano, nitro, hydroxy, des groupes alkyle, alcoxy, dioxyalkylène, alkylcarbonyloxy et alkylthio chacun à chaîne droite ou ramifiée et contenant chacun jusqu'à 4 atomes de carbone, des groupes halogénoalkyle, halogénoalcoxy ou halogénoalkylthio à chaîne droite ou ramifiée contenant chacun jusqu'à 4 atomes de carbone et jusqu'à 9 atomes d'halogènes identiques ou différents ou des groupes phényle, et

Het représente un hétérocycle portant éventuellement 1 à 3 substituants identiques ou différents et/ou condensé sur un noyau benzénique, de formule

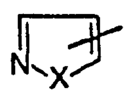 ; 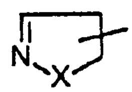 ; 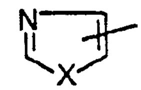 ;

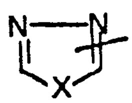 ; 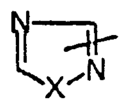 ; 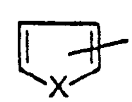 ;

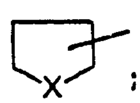 ; 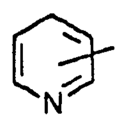 ; 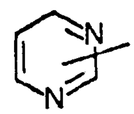 ;

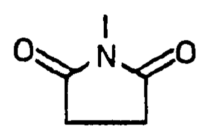 ou 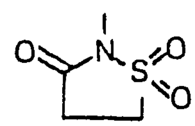

les substituants en question étant: le fluor, le chlore, le brome, des groupes méthyle, éthyle, n- ou isopropyle, n-, iso-, sec- ou tert-butyle, méthoxy, éthoxy, trifluorométhyle ou des groupes phényle portant éventuellement 1 à 3 substituants identiques ou différents choisis parmi le chlore, les groupes nitro, méthyle et/ou méthoxy, et

X représente dans chaque cas l'oxygène ou le soufre,

caractérisé en ce que l'on fait réagir des esters d'acides hydroximinocarboxyliques de formule XI

```
     O  O
     ||  ||
R¹-C-C-C-OR        (XI)
        ||
        N-OH
```

dans laquelle

R et $R^1$ ont les significations indiquées ci-dessus, avec des agents alkylants de formule III

$$Het\text{-}CH_2\text{-}X' \qquad (III)$$

dans laquelle

Het a les significations indiquées ci-dessus, et
X' représente un groupe éliminable électrophile,

éventuellement en présence d'un diluant et éventuellement en présence d'un diluant et éventuellement en présence d'un capteur d'acide, à des températures allant de +10 à +80°C.